# EUROPEAN PATENT SPECIFICATION

(11) **EP 2 873 726 B1**
(45) Date of publication and mention of the grant of the patent: **05.09.2018**
(21) Application number: 13816104.7
(22) Date of filing: 12.07.2013
(51) Int. Cl.: C12N 1/21, A61P 35/00, C07K 19/00, C07K 16/28, C07K 16/30, A61K 35/74, A61K 39/02, A61K 48/00, C12N 15/09, C12Q 1/04

(54) **ANTITUMOR AGENT, MARKER FOR TUMOR DETECTION, AND ORAL VACCINE AGENT**
ANTITUMORMITTEL, MARKER FÜR TUMORNACHWEIS UND ORALER IMPFSTOFF
AGENT ANTITUMORAL, MARQUEUR DE DÉTECTION DE TUMEUR, ET AGENT VACCINAL À USAGE ORAL

(30) Priority: 13.07.2012 JP 2012158044
(43) Date of publication of application: 20.05.2015
(73) Proprietor: Teikyo Heisei University, Tokyo 170-8445 (JP)
(72) Inventor: TAIRA, Yuichiro, Tokyo 107-0052 (JP); ISHIDA, Isao, Isehara-shi Kanagawa 259-1136 (JP)
(74) Representative: Denison, Christopher Marcus
(86) International application number: PCT/JP2013/069717
(87) International publication number: WO 2014/010758

(56) References cited:
- EP-A1- 2 283 810
- WO-A1-00/06764
- WO-A1-2010/126073
- WO-A1-2010/126073
- WO-A1-2011/005756
- GB-A- 2 482 535
- JP-A- 2005 523 875
- GROOT ET AL: "Functional antibodies produced by oncolytic clostridia", BIOCHEMICAL AND BIOPHYSICAL RESEARCH COMMUNICATIONS, ACADEMIC PRESS INC. ORLANDO, FL, US, vol. 364, no. 4, 29 October 2007 (2007-10-29), pages 985-989, XP022344222, ISSN: 0006-291X, DOI: 10.1016/J.BBRC.2007.10.126
- MICHL P ET AL: "Bacteria and bacterial toxins as therapeutic agents for solid tumors", CURRENT CANCER DRUG TARGETS, BENTHAM SCIENCE PUBLISHERS, HILVERSUM, NL, vol. 4, no. 8, 1 December 2004 (2004-12-01), pages 689-702, XP009101385, ISSN: 1568-0096, DOI: 10.2174/1568009043332727
- CRONIN MICHELLE ET AL: "Orally Administered Bifidobacteria as Vehicles for Delivery of Agents to Systemic Tumors", MOLECULAR THERAPY, NATURE PUBLISHING GROUP, GB, vol. 18, no. 7, 1 July 2010 (2010-07-01), pages 1397-1407, XP009141815, ISSN: 1525-0016, DOI: 10.1038/MT.2010.59
- CATHERINE A KING ET AL: "DNA vaccines with single-chain Fv fused to fragment C of tetanus toxi induce protective immunity against lymphoma and myeloma", NATURE MEDICINE, NATURE PUBLISHING GROUP, NEW YORK, NY, US, vol. 4, no. 11, 1 November 1998 (1998-11-01), pages 1281-1286, XP002173090, ISSN: 1078-8956, DOI: 10.1038/3266
- BJÖRN LÖWENADLER ET AL: "A gene fusion system for generating antibodies against short peptides", GENE, ELSEVIER, AMSTERDAM, NL, vol. 58, no. 1, 1 January 1987 (1987-01-01), pages 87-97, XP001313276, ISSN: 0378-1119
- RUSH C ET AL: "Protein A as a fusion partner for the expression of heterologous proteins in Lactobacillus", APPLIED MICROBIOLOGY AND BIOTECHNOLOGY, SPRINGER, DE, vol. 47, no. 5, 1 January 1997 (1997-01-01), pages 537-542, XP002209512, ISSN: 0175-7598, DOI: 10.1007/S002530050969
- TANIGUCHI S. ET AL.: 'Targeting solid tumors with non-pathogenic obligate anaerobic bacteria' CANCER SCI. vol. 101, no. 9, 2010, pages 1925 - 1932, XP055184119

## Description

### Technical Field

The present invention relates to an antitumor agent, and an agent for tumor detection, each containing a recombinant *Bifidobacterium* as an active ingredient.

### Background Art

Obligate anaerobes, including *Bifidobacterium,* grow only in tumors with very high selectivity when intravenously administered (Non-patent Document 1). This may be because the central region of a tumor is hypoxic; thus, there is an anaerobic environment which allows obligate anaerobes to survive and grow. Moreover, *Bifidobacterium* is a gram-positive bacterium, which is advantageous in that it has no risk of releasing any endotoxin after death. Meanwhile, the existence of such an anaerobic environment at the central region of the tumor tissue is thought to cause resistance against conventional chemotherapy (Non-patent Document 2). Therefore, the usefulness of *Bifidobacterium* as a DDS (drug delivery system) carrier for tumor tissue employing the above properties of *Bifidobacterium* is currently attracting attention.

For example, Patent Document 1 and Non-patent Document 3 disclose an invention for treating solid cancer, which comprises preparing recombinant *Bifidobacterium* that expresses *Escherichia coli* cytosine deaminase (CD) capable of converting a prodrug, 5-fluorocytosine (5-FC) to 5-fluorouracil (5-FU), and administering the recombinant *Bifidobacterium* with 5-FC, so as to obtain antitumor effects. However, this method is problematic in that it is complicated since it requires administering *in vivo* a large amount of 5-FC in addition to the recombinant *Bifidobacterium,* thereby imposing various constraints upon clinical development in view of side effects, etc.

Moreover, Patent Document 2 discloses an invention that comprises preparing recombinant *Bifidobactrium* capable of high-level secretory expression of a biologically active polypeptide, and directly injecting the recombinant *Bifidobacterium* into a tumor. However, this method is problematic in that the position of the relevant tumor is required to be precisely specified, resulting in a narrow application range, and it is highly invasive when a tumor is present in the deep interior of the body, thereby lacking general versatility.

As methods for treating tumors using other bacteria, for example, methods using *Clostridium novyi* described in Patent Document 3 and Non-patent Documents 4 and 5, and methods using *Salmonella typhimurium* described in Non-patent Documents 6 and 7 have been reported. All of these bacteria are derived from pathogenic bacteria and secrete harmful exotoxins, and thus they pose serious problems to the safety of the living body to which they are administered. Michl and Gress, Current Cancer Drug Targets, 2004, 4, 689-702 provides a review of bacteria and bacterial toxins as therapeutic agents for solid tumours.

### Prior Art Documents

### Patent Documents

Patent Document 1: JP Patent No. 3642755
Patent Document 2: WO/2010/126073
Patent Document 3: JP Patent Publication (Kokai) No. 2009-541319 A

### Non-patent Documents

Non-patent Document 1: Kimura N.T., et al., 1980, Cancer Res., 40 (6): 2061-2068.
Non-patent Document 2: Tredan O, et al., 2007, J Natl Cancer Inst, 99: 1441-1454.
Non-patent Document 3: S. Taniguchi et al., 2010, Cancer. Sci., 101 (9): 1925.
Non-patent Document 4: Dang L.H., et al., 2001, Proc. Natl. Acad. Sci. U.S.A., 98: 15155-15160.
Non-patent Document 5: Ian C., et al., 2006, Science 314: 1308-1311.
Non-patent Document 6: Pawelek J.M., et al., 1997, Cancer Res., 57:4537-4544.
Non-patent Document 7: Toso J.F., et al., 2002, J. Clin. Oncol., 20:142-152.

### Summary of the Invention

An object of the present invention is to provide a recombinant obligate anaerobic gram-positive bacterium functioning as a drug delivery carrier capable of delivering a functional peptide-encoding nucleic acid to the surrounding areas of target cells in an expressible state.

An object of the present invention is to provide an antitumor agent with low invasiveness, few side effects, and high target specificity and/or a marker for tumor detection allowing monitoring therapeutic effects over time.

Also described herein is a noninvasive oral vaccine agent with few side effects and high target specificity.

In view of the clinical application of an obligate anaerobe as a drug delivery carrier, a therapeutic method that involves highly versatile intravenous administration is effective. Intravenous administration requires specific targeting of cancer cells and construction of a biologically active peptide with high cellular cytotoxicity. However, there is a big problem that a functional antibody is not expressed when immunoglobulin (IgG) is expressed in *Bifidobacterium.*

To solve the above problems, the present inventors prepared recombinant (genetically modified) *Bifidobacterium* containing a nucleic acid encoding a secretory fusion protein comprising a signal peptide, a low-molecular-weight antitumor antibody composed of a V_{HH} antibody of the family *Camelidae'* and an exotoxin, and then intravenously administered the recombinant *Bifidobacterium* to a cancer-bearing Xenograft mouse model. The recombinant *Bifidobacterium* was accumulated within the tumor with very high selectivity, and it successfully suppressed tumor cell proliferation by 90% or more due to the cellular cytotoxicity of the secreted fusion protein without causing any severe side effects on the living body. The present invention is based on the study results. Specifically, the following invention is provided.
(1) A recombinant Bifidobacterium, comprising a nucleic acid encoding a fusion protein comprising a signal peptide, one or more V_{HH} antibodies of the family Camelidae, and one or more functional proteins selected from exotoxins, labeling proteins, enzymatic labels, or a combination thereof, in a secretable state, wherein the V_{HH} antibodies of the family Camelidae recognize and bind to the surface antigen of a tumor cell.
(2) The recombinant Bifidobacterium according to (1), wherein the nucleic acid encodes 2 or more VHH antibodies of the family Camelidae.
(3) The recombinant Bifidobacterium according to (1 or 2), wherein the labeling protein is a fluorescent protein or a luminescent protein.
(4) The recombinant Bifidobacterium according to any one of 1-3, for use as an antitumor agent.

The invention also provides the recombinant Bifidobacterium according to anyone of 1 to 3, for use in vivo for tumor detection.

### Brief Description of the Drawings

Fig. 1 shows the structure of each expression cassette encoding a fusion protein used in experiments.
Fig. 2 shows the immunoblotting of the constructed fusion proteins.
Fig. 3A shows the inhibition of A431 cell proliferation by each fusion protein.
Fig. 3B shows the inhibition of BxPC-3 cell proliferation by each fusion protein.
Fig. 3C shows the inhibition of HT-29 cell proliferation by each fusion protein.
Fig. 4A shows the binding of A431 cells to Ec-8C7EGFP. The left columns show the results of a negative control to which only a buffer was added. The right columns show the results when Ec-8C7EGFP was added. Each upper column shows a fluorescent image, and each lower column shows a bright-field image of the same field of view.
Fig. 4B shows the binding of HT-29 cells to Ec-8C7EGFP. The left columns show the results of a negative control to which only a buffer was added. The right columns show the results when Ec-8C7EGFP was added. Each upper column shows a fluorescence image, and each lower column shows a bright-field image of the same field of view.
Fig. 4C shows the results over time of EGFR internalization in A431 cells. The upper columns show fluorescence images and the lower columns show bright-field images of the same field of view.
Fig. 5 shows binding affinity between Ec-8C7 EGFP and recombinant human EGFR extracellular domain.
Fig. 6 shows the structure of a shuttle vector pKKT427 of *Bifidobacterium* and *Escherichia coli.*
Fig. 7 shows the number of colonies of 8C7-toxin-expressing recombinant *Bifidobacterium* that appeared on an MRS selective agar medium. "Tumor," "Blood," and "Spleen" denote samples collected from cancer-bearing mice to which human skin cancer-derived cells (A431) were subcutaneously transplanted. A white circle, a black circle, a triangle, etc., for each sample indicate that these are derived from the same individual mouse. A horizontal bar for each sample denotes an average value.
Fig. 8 shows changes in liver functions of cancer-bearing mice to which A431 cells had been subcutaneously transplanted, when 8C7-toxin-expressing recombinant *Bifidobacterium* was intravenously administered to the mice (n=5).
Fig. 9 shows the EGFR internalization of 8C7 EGFP secreted by recombinant *Bifidobacterium* in cancer-bearing mice to which A431 cells had been transplanted.
Fig. 10 shows antitumor effects exerted when 8C7-toxin-expressing recombinant *Bifidobacterium* was intravenously administered to cancer-bearing mice to which A431 cells had been subcutaneously transplanted.
Fig. 11 shows antitumor effects exerted when 8C7-toxin-expressing recombinant *Bifidobacterium* was intravenously administered to cancer-bearing mice to which BxPC-3 cells had been subcutaneously transplanted.
Fig. 12 shows antitumor effects exerted when 8C7-toxin-expressing recombinant *Bifidobacterium* was intravenously administered to cancer-bearing mice to which human non-small-cell lung cancer-derived PC-9 cells had been subcutaneously transplanted.
Fig. 13 shows anti-EGFP antibody production in feces of each mouse (8C7 EGFP) to which 8C7-EGFP-expressing recombinant *Bifidobacterium* was administered orally and each negative control mouse (pKKT427) to which pKKT427-containing recombinant *Bifidobacterium* was administered orally.
Fig. 14 shows the induction of an anti-8C7 EGFP antibody in mouse serum when 8C7-toxin-expressing recombinant *Bifidobacterium* was intravenously administered to cancer-bearing normal mice to which colon 26 cells had been subcutaneously transplanted. "nIgG" denotes negative control Normal Mouse IgG. "Anti-EGFP antibody" is a positive control.

### Modes for Carrying Out the Invention

The present invention will be described below in detail.

### 1. Recombinant obligate anaerobic gram-positive bacterium

### 1-1. Overview and definition

A 1^{st} aspect of the present invention is a recombinant obligate anaerobic gram-positive Bifidobacterium (hereinafter, often abbreviated as "recombinant bacteria (bacterium)") as defined in the claims. The recombinant bacterium of the present invention is a drug delivery carrier containing a nucleic acid encoding a fusion protein in an expressible state.

The term "obligate anaerobic gram-positive bacteria (bacterium)" refers to obligate anaerobes that are classified as gram-positive bacteria. Herein, the term "obligate anaerobes" refers to bacteria that are also referred to as "strictly/obligately anaerobic bacteria" and cannot grow and die in the presence of high oxygen concentrations. Therefore, in a mammalian body, these bacteria can grow in a low-oxygen anaerobic state, such as within digestive organs and mainly in the intestine, but cannot grow in body fluids such as blood in which dissolved oxygen is present and in general tissues. The term "gram-positive bacteria (bacterium)" refers to the generic name of bacteria that are stained violet or dark blue by Gram staining. Bacilli, cocci, and spirilli are known examples of gram-positive bacteria. Gram-positive bacteria contain no endotoxin, and thus release no endotoxin after death. Therefore, gram-positive bacteria are preferable as drug delivery carriers in terms of safety. The recombinant bacterium of the present invention belongs to the genus *Bifidobacterium* (hereinafter "the genus *Bifidobacterium"* is referred to as *"Bifidobacterium"* as a generic name).. This is because *Bifidobacterium* does not secrete exotoxin, is daily used as lactic acid bacteria, and has been confirmed to be safe, for example for human bodies. *Bifidobacterium* may be of any species. Preferable examples thereof are species that inhabit human intestine, in particular, *B. bifidum, B. longum, B. brave, B. infantis,* and *B. adolescentis.*

### 1-2. Configuration

The obligate anaerobic gram-positive bacterium of the present invention contains a nucleic acid (hereinafter, often referred to as "fusion gene") encoding a fusion protein in an expressible state. Hereinafter, the fusion gene, by which the recombinant bacterium of the present invention is characterized, and the configuration of an expression cassette that enables the expression of the fusion gene are specifically explained.

### 1-2-1. Configuration of fusion gene

The term "nucleic acid (fusion gene) encoding a fusion protein" as used herein refers to a fusion-protein-encoding foreign nucleic acid that is constructed by the fusion of a plurality of genes, etc., according to gene-recombination technology. The fusion gene is introduced into the recombinant bacteria of the present invention, in a state of being inserted within an expression cassette described later.

The term "fusion protein" as used herein refers to an extracellular secretory protein containing signal peptide(s), single-chain antibody(ies), and functional peptide(s), which are linked. The signal peptide(s), the single-chain antibody(ies) and the functional peptide(s) may be directly linked or indirectly linked via linker peptides. The length and the amino acid sequence of a linker peptide are not particularly limited, as long as it does not inhibit the respective functions of the single-chain antibody and the functional peptide. A preferred example thereof is an amino acid sequence that has a length of 20 amino acids or less or 15 amino acids or less and is not self-folded. Hereinafter, such a signal peptide, single-chain antibody(ies), and functional peptide(s) that compose a fusion protein are specifically explained.

### (1) Signal peptide

A signal peptide is required for the extracellular transfer of proteins that are biosynthesized within cells. In general, positively charged amino acids such as Lys and Arg are placed on the N-terminal side, after which highly hydrophobic amino acids such as Ala, Leu, Val, Ile, Val, and Phe are placed. Moreover, the signal peptide may contain, on the C-terminal side thereof, an insertion sequence (following the signal sequence) which facilitates the cleavage and secretion of the signal peptide and/or an amino acid sequence comprising a site recognized by signal peptidase cleaving the signal peptide from the fusion protein,. The signal peptide plays a role in extracellularly secreting the fusion protein that is expressed within the bacteria of the present invention via a translocator or the like that exists in the membrane. The amino acid sequence of the signal peptide is not particularly limited. The amino acid sequences of any known signal sequences capable of functioning within obligate anaerobic gram-positive bacteria can be used herein. The amino acid length of a signal peptide is also not particularly limited. In general, the length may range from 3 to 60 amino acids. A signal peptide with a short amino acid length is preferred so that the molecular weight of the fusion protein is not too large.

The signal peptide is placed on the N-terminal side of the fusion protein.

### (2) Single-chain antibody

The above fusion protein contains single-chain antibody(ies). The term "single-chain antibody" as used herein refers to an antibody that is composed of a single-chain polypeptide, and can recognize and bind to a target substance alone. This is because an antibody composed of two or more chains is too large in terms of molecular weight, and thus such an antibody is unlikely expressed within obligate anaerobic gram-positive bacteria and an appropriate three dimensional structure having antibody functions is unlikely constructed. A single-chain antibody may be either a natural antibody or an artificial antibody.

The term "natural antibody" as used herein refers to an antibody having the same amino acid sequence as that of an antibody that is produced by any vertebrates. A specific example of a natural single-chain antibody is a single-chain antibody that is produced by animals of the family Camelidae (Zhang L., et al., 1993, Nature, 362: 446-448) (hereinafter, referred to as "single-chain antibody of the family Camelidae"). The single-chain antibody of the family Camelidae is composed of H chain alone (without L chain), is capable of binding to an antigen with the V_{H} region alone of its H chain, and thus the V_{H} region has a molecular weight of about 14 kDa that is only about one tenth the molecular weight of a general antibody. The single chain antibody used in the present invention is a V_{HH} antibody. Moreover, the single-chain antibody of the family Camelidae generally has high antigen affinity, and is characterized by high resistance to heat, acid, and base (Deffar, K., et al., 2009, African Journal of Biotechnology 8 (12): 2645-2652). Therefore, the V_{HH} single-chain antibody of the family Camelidae is used as a V_{HH} single-chain antibody in the present invention. Any animal species of the family Camelidae can be used herein, as long as it can produce the single-chain antibody of the family Camelidae. For example, antibodies from any animal species such as lamas, alpacas, and camels can be used.

The term "artificial antibody" as used herein refers to an artificially constructed antibody. Examples thereof include, in addition to a single-chain antibody prepared by introducing an appropriate mutation into the amino acid sequence of the afore-mentioned natural antibody, and a structurally modified single-chain antibody that does not exist in principle in the nature.

The above fusion protein may contain 2 or more V_{HH} antibodies of the family Camelidae. However, the higher the number of antibodies, the larger the molecular weight of the fusion protein. Hence, the preferable number of single-chain antibodies is several such as 2 to 4, or 2 to 3, or 2. Individual single-chain antibodies are desirably linked via appropriate linker peptide(s). The length and the amino acid sequence of a linker peptide are not particularly limited, as long as it does not inhibit the antigen binding activity of each single-chain antibody. For example, such a linker peptide may be identical to a linker peptide that links the above-mentioned single-chain antibody and an exotoxin. When the fusion protein contains 2 or more single-chain antibodies, individual single-chain antibodies may recognize different antigen epitopes. A fusion protein containing 2 or more single-chain antibodies that recognize different epitopes of the same target substance as an antigen may have enhanced antigen affinity.

A single-chain antibody according to the present invention recognizes and binds to a surface antigen on a tumor cell. As described above, the recombinant bacterium of the present invention can grow only in an anaerobic environment. Hence, tumour cells in an anaerobic environment *in vivo* are suitable target cells.

The order of single-chain antibodies and functional peptides described later is not particularly limited, as long as the antibodies are placed on the C-terminal side of a signal peptide in a fusion protein. Single-chain antibodies are preferably placed on the N-terminal side of functional peptides.

### (3) Functional peptide

The above fusion protein contains functional peptide(s) selected from an exotoxin and a label selected from labelling proteins and enzymatic labels. The term "functional peptide" in general as used herein refers to a peptide having, in a living body or within cells, specific bioactivity such as enzymatic activity, catalytic activity, functions as a substrate, or biological inhibitory or enhancement activity (for example, cellular cytotoxicity). Specific examples thereof include an exotoxin, a fluorescent protein, or a luminescent protein, an enzyme, and an antigen peptide.

Functional peptides may be derived from any biological species. Moreover, the functional peptides may be either natural or unnatural. The term "natural functional polypeptide" refers to a peptide that exists in the nature. On the other hand, the term "unnatural functional polypeptide" refers to a modified peptide prepared by, on the basis of the amino acid sequence of a natural functional polypeptide, introducing an appropriate mutation (such as addition, deletion, and/or substitution of an amino acid(s)) into the amino acid sequence, as long as the functional peptide does not lose its own unique functions.

The above fusion protein may contain 2 or more functional peptides. However, when the above fusion protein contains a plurality of functional peptides, the overall molecular weight of the fusion protein is too large. The total molecular weight of the functional peptides is preferably 80 kDa or less, and preferably 40 kDa or less. When the above fusion protein contains 2 or more functional peptides, functional peptides may be of the same type or different types. Examples thereof include a functional peptide comprising an exotoxin combined with an enzyme, or an exotoxin combined with a fluorescent protein or a luminescent protein. When the above fusion protein contains 2 or more functional peptides, individual functional peptides may be directly linked and preferably linked via appropriate linker peptide(s) so that each functional peptide can efficiently exhibit its unique functions. The length and the amino acid sequence of a linker peptide are not particularly limited, as long as it does not inhibit the functions of the functional peptide. Two or more different functional peptides are contained in one fusion protein, so that the fusion protein has different functions to a target substance recognized by a single-chain antibody.

The functional peptides are preferably placed on the C-terminal side of the above single-chain antibodies without being particularly limited, as long as it is located on the C-terminal side of a signal peptide in a fusion protein.

Hereinafter, the above-described exotoxin, enzyme, fluorescent protein or luminescent protein, and antigen peptide, which can function as functional peptides, are specifically explained.

### (i) Exotoxin

The term "exotoxin" refers to a toxic protein that is secreted outside the cells by bacteria. An exotoxin to be used herein may be of any type as long as it has cellular cytotoxicity and the nucleotide sequence thereof is known. Examples of an exotoxin include *Pseudomonas aeruginosa* toxin (Pseudomonas toxin; PT) and derivatives thereof such as exotoxin A (SEQ ID NO: 9) prepared by removing a cell adhesion domain from PT, Diphtheria toxin (DT) and derivatives thereof, and Ricin and derivatives thereof (Brinkmann U. & Pastan I., 1994, Biochimica et Biophysica Acta, 1198 (1): 27-45). *Pseudomonas aeruginosa* exotoxin A is known to inhibit protein synthesis by inactivating EF-2 by ADP ribosylation and to exhibit strong cytotoxic effect, after incorporation thereof into cancer cells. A fusion protein in which an exotoxin is linked to the above single-chain antibody can function as an immunotoxin.

### (ii) Enzyme

The type of an enzyme as a functional peptide is not particularly limited, as long as the nucleotide sequence thereof is known. Such an enzyme may be an enzyme that directly acts on a target substance or an enzyme that does not directly act on, but acts around a target substance. Specific examples of the latter enzyme include luciferase that contributes to luminescence, and peroxidase (e.g., horseradish peroxidase). A fusion protein in which such an enzyme is linked to the above single-chain antibody can function as an immunoenzyme.

### (iii) Fluorescent protein or luminescent protein (labeling protein)

The type of a fluorescent protein as a functional peptide is not particularly limited, as long as the nucleotide sequence thereof is known. Such a fluorescent protein may be either natural or unnatural. Because of the above reason, a fluorescent protein having a short amino acid length is preferred. Moreover, excitation wavelength and fluorescence wavelength are not particularly limited. These wavelengths may be adequately selected according to the situation and need. Specific examples of the fluorescent protein include CFP, RFP, DsRed, YFP, PE, PerCP, APC, and GFP.

Furthermore, the type of a luminescent protein is also not particularly limited, as long as the nucleotide sequence thereof is known. Similarly to the above fluorescent protein, a luminescent protein may be either natural or unnatural, and a luminescent protein having a short amino acid length is preferred. A specific example of the luminescent protein is aequorin.

### (iv) Antigen peptide

The term "antigen peptide" as used herein refers to a peptide that contains 1 or more epitopes (antigenic determinants) and can induce an immune response in an individual to which the peptide has been administered. The type of an antigen peptide is not particularly limited, as long as it has activity to induce the above immune response; that is, contains 1 or more antigen epitopes. Examples of the antigen peptide include peptides derived from viruses (influenza virus, hepatitis C virus, hepatitis E virus, herpes simplex virus, human T-cell leukemia virus type 1, AIDS virus, West Nile fever virus, papilloma virus, rotavirus, and Norwalk virus), and peptides derived from HER2 protein, Plasmodium (protozoan), Trypanosoma (protozoan), and *Salmonella typhi.* However, this is based on the premise that the peptide antigen is not recognized by a single-chain antibody in a fusion protein. The amino acid length of the antigen peptide is also not particularly limited, as long as it contains 1 or more epitopes. However, since the total molecular weight of functional peptides is limited to some extent as described above, the upper limit is a length of 800 amino acids or less and preferably 400 amino acids or less. Furthermore, the lower limit is a minimum amino acid length that allows the antigen peptide to contain 1 epitope, such as 7 or more amino acids, preferably 8 or more amino acids, and more preferably 9 or more amino acids. A fusion protein, in which an antigen peptide is linked to the above single-chain antibody, can function as an immunoantigen (immune antigen; fusion peptide of an antibody and an antigen).

### 1-2-2. Configuration of expression cassette

The term "expression cassette" as used herein refers to an expression system that contains the above fusion gene and enables the fusion gene to be expressed as a fusion protein. The phrase a "expressible state" means that a fusion gene is placed under the control of elements required for gene expression so that the fusion gene contained in the expression cassette can be expressed within recombinant bacteria. Examples of elements required for gene expression include a promoter and a terminator.

Promoters to be used herein are not particularly limited, as long as these promoters can be activated within recombinant bacteria. Promoters derived from obligate anaerobic gram-positive bacteria to be used are preferred. When *B. longum* (*Bifidobacterium*) is used as an obligate anaerobic gram-positive bacterium, an example thereof is the hup gene promoter (SEQ ID NO: 4) of *B. longum.* Furthermore, promoters differing in their properties of expression control including an overexpression promoter, a constitutive promoter, a site-specific promoter, a stage-specific promoter, an inducible promoter, and the like are known. A promoter to be used for an expression cassette in the present invention may be any promoter and is not particularly limited. A promoter may be adequately selected as necessary. A preferable example thereof is an overexpression promoter or a constitutive promoter. In the above expression cassette, a promoter is placed on the 5' side and upstream of the initiation codon of the above fusion gene.

A terminator to be used herein is not particularly limited, as long as it can terminate the transcription of a gene transcribed by the above promoter within recombinant bacteria. An example of such a terminator is the histone-like protein terminator (HUT) (SEQ ID NO: 10). A terminator to be used herein is preferably a terminator derived from the same biological species as that of a promoter, and is more preferably a terminator that forms a pair with a promoter on the genome of the biological species from which the promoter is derived. In the above expression cassette, a terminator is placed on the 3 'side and downstream of the termination codon of the above fusion gene.

To stably express the above fusion protein within recombinant bacteria, a vector containing an expression cassette can be introduced as an expression vector into the cells. Alternatively, a vector may be inserted into the genome of the cells via homologous recombination. When an expression vector is used, a plasmid or the like can be used as a vector. A vector to be used herein is replicable within the recombinant bacterium of the present invention and contains an appropriate selection marker gene that is stably retained within cells. A vector may be a shuttle vector that is replicable within other bacteria such as *Escherichia coli.* Examples thereof include pKKT427, pBESAF2, and pPSAB1. When a vector is inserted into the genome of recombinant bacteria, a fusion gene alone may be inserted into the genome of recombinant bacteria in an expressible state. Specifically, a fusion gene may be inserted under the control of an endogenous promoter and/or an endogenous terminator of recombinant bacteria.

An expression cassette may be monocistronic, which contains one fusion gene within one expression cassette, or polycistronic, which contains 2 or more fusion genes. 1-3. Method for producing recombinant obligate anaerobic gram-positive bacterium

The recombinant bacterium of the present invention can be produced using molecular genetic methods known in the art. For example, in the case of the above fusion gene, a nucleic acid encoding each of a signal peptide, a single-chain antibody, and an exotoxin is constructed, for example, using techniques described in Sambrook et al., Molecular Cloning, 3rd Ed., Current Protocols in Molecular Biology (2001), Cold Spring Harbor Laboratory Press or Ausubel et al., Short Protocols in Molecular Biology, 3rd Ed., A compendium of Methods from Current Protocols in Molecular Biology (1995), John Wiley & Sons.

An antigen for the above single-chain antibody may be any surface antigen of a tumor cell. However, in this case, the nucleotide sequence information of a gene encoding an antibody that specifically recognizes and binds to such a target substance is required. If the nucleotide sequence of an antibody against a target substance is known, the sequence information thereof can be used.

When an antibody against a target substance is unknown, a monoclonal antibody against the target substance can be prepared according to a method known in the art. A preparation example thereof is as follows.

Target tumor cells or the like are administered as immunogens to animals of the family Camelidae for immunization. If necessary, an adjuvant may be added for effective immunization. Examples of an adjuvant include commercially available complete Freund's adjuvant (FCA) and incomplete Freund's adjuvant (FIA), and these adjuvants can be used alone or in combination. A single dose of an immunogen solution may contain about 50 µg to 200 µg of the immunogen per animal above. The intervals for immunization are not particularly limited. After primary immunization, an immunized animal is boosted 2 to 10 times and preferably 5 to 7 times at intervals of several days to several weeks, and preferably 1 to 4 weeks. After primary immunization, antibody titer in the serum of the immunized animal is measured repeatedly by ELISA (Enzyme-Linked Immuno Sorbent Assay), for example. Subsequently, antibody-producing cells are collected from the immunized animal. Examples of antibody-producing cells include spleen cells, lymph node cells, and peripheral blood cells, and are preferably peripheral blood cells. Next, RNA is extracted from peripheral blood cells, and then cDNA is synthesized using an oligo dT primer and a random 6 mer primer. From the cDNA, the gene of the variable region (V_{HH} region) of the single-chain antibody of the family Camelidae is amplified by PCR, the above gene is incorporated into a phagemid vector such as pCANTAB6, and then the vector is introduced into *Escherichia coli* TG1 by electroporation. The above *Escherichia coli* TG1 is infected with an M13K07 helper phage, the phage is collected, and thus a single-chain antibody variable region (V_{HH} region) of the family Camelidae expression phage library is obtained. This is normally a library of 1 × 10⁷ pfu or more.

Biopanning is performed to select phages expressing an antibody against a target antigen. Biopanning is a method for concentrating a phage specific to a target antigen, which involves reacting an antibody phage library with an immobilized target antigen, removing unbound phages by washing, eluting phages binding to the target antigen, infecting *Escherichia coli* with the phages for proliferation, and repeating these procedures 3 to 5 times. After re-infection of *Escherichia coli* TG1 with phages subjected to biopanning, TG1 clones containing V_{HH} insertion pCANTAB phagemid vector are isolated. TG1 clones are each infected with a KO7 helper phage, and thus cloned phages presenting the V_{HH} antibody are obtained. Clones that react with the antigen are selected from among these phages. The nucleotide sequence of the antibody can be obtained from the thus obtained phages.

A fusion gene is inserted into the above expression cassette using a molecular genetic method so that the fusion gene can be expressed. An expression cassette is incorporated into a vector such as a plasmid, as necessary. An expression cassette may be incorporated into a vector, for example, using a method that involves cleaving the 5'end and the 3'end of an expression cassette with appropriate restriction enzyme(s), and then inserting it into a corresponding restriction site such as a multicloning site within the vector. Moreover, when a vector is an expression vector that enables expression within the recombinant bacterium of the present invention, the above fusion gene is inserted into the expression control region (e.g., a multicloning site between a promoter and a terminator within the vector) of the expression vector, and thus the expression cassette and the target expression vector can be constructed at the same time. Regarding these specific methods, for example, the method described in the above Sambrook et al. (2001) may be referred to.

Recombinant bacteria of interest can be produced by introducing the above expression vector, expression cassette, or fusion gene into obligate anaerobic gram-positive bacteria as drug delivery carriers. As a method for introducing an expression vector or the like into target obligate anaerobic gram-positive bacteria, a molecular biological method known in the art can be employed. For example, a known method such as electroporation or a calcium phosphate method can be employed. Regarding these specific methods, for example, the method described in the above Sambrook et al., (2001) may be referred to.

### 2. Antitumor agent

### 2-1. Overview

A 2^{nd} aspect of the present invention is the recombinant Bifidobacterium for use as an antitumor agent. The term "antitumor agent" in the present invention refers to a drug having cellular cytotoxicity against tumor cells, causing apoptosis of the cells and as a result, being capable of suppressing the proliferation of the tumor cells. The antitumor agent is required to have a beneficial effect of suppressing tumor cell proliferation, but is not required to eradicate tumor cells.

The antitumor agent of the present invention is characterized by containing the recombinant bacterium of the 1^{st} aspect as an active ingredient. According to the antitumor agent of the present invention, the recombinant bacterium as an active ingredient grows only within tumors and secrets immunotoxin within tumors, and thus can efficiently suppress tumor growth and can cause tumor regression.

### 2-2. Configuration

The antitumor agent of the present invention contains the recombinant bacterium of the 1^{st} aspect as an active ingredient as described above. The recombinant bacterium in this case is characterized in that the fusion gene encodes a VHH antibody of the family Camelidae recognizing and binding to a surface antigen of target tumor cells, and, a functional peptide encodes an exotoxin. Therefore, the recombinant bacterium as an active ingredient of the antitumor agent of the present invention secretes an extracellular secretory antitumor cell immunotoxin.

In general, a tumor cell expresses cancer cell surface antigen(s) on the cell surface. An example of such an antigen is a carcinoembryonic antigen and Mesothelin. A single-chain antibody encoded by a fusion gene preferably recognizes and binds to such surface antigens. Alternatively, when cells become cancerous due to functional enhancement of a receptor that controls cell proliferation, such as EGFR (epidermal growth factor receptor) (in the case of EGFR exemplified herein, non-small-cell lung cancer, prostate cancer, and the like), a single-chain antibody that can be used herein recognizes and binds to an extracellular domain of a receptor or the like that causes the oncogenic transformation (in the above example, EGFR). In this manner, the recombinant bacterium as an active ingredient can cope with any tumor antigens through exchange of the nucleotide sequence of a single-chain antibody moiety in a fusion gene encoding an extracellular secretory immunotoxin. Tumors targeted by the antitumor agent of the present invention may be any tumors that are formed, regardless of benign and malignancy. Examples of target tumors include brain tumor, thyroid cancer, oral cancer, esophageal cancer, gastric cancer, large bowel cancer, pharyngeal cancer, lung cancer, liver cancer, renal cancer, adrenal cancer, pancreatic cancer, biliary tract cancer, cervical cancer, cancer of uterine body, ovarian carinoma, mammary cancer, prostate cancer, bladder cancer, fibrosarcoma, mastocytoma, and melanoma.

When the recombinant bacterium of the present invention expresses a fusion gene, its product, extracellular secretory antitumor cell immunotoxin, is secreted extracellularly. The secreted immunotoxin binds to tumor cells as target substances, with its single-chain antibody moiety, so that apoptosis of the cells is induced because of the cellular cytotoxicity of the exotoxin moiety.

The recombinant bacterium of the present invention, as an active ingredient, is contained in a living state in the antitumor agent. The recombinant bacterium described in the 1^{st} aspect of the present invention cannot grow in the presence of high oxygen concentrations and then die. Hence, in a living body, the recombinant bacterium can grow and survive at only sites with low oxygen partial pressure. Typical examples of such a site include central regions of tumors (solid cancer) observed for advanced cancer or within the intestine. Therefore, the antitumor agent of the present invention can be an antitumor agent delivered to tumors with high selectivity when administered *in vivo* by injection or the like.

Furthermore, the antitumor agent of the present invention can be used in combination with other antitumor agents as long as the survival and the growth of the recombinant bacterium as an active ingredient, and the expression and the secretion of an immunotoxin are not inhibited or suppressed.

The antitumor agent of the present invention can be formulated in principle by a method known in the art on the assumption that the recombinant bacterium is maintained or preserved in a living state. For example, the method described in Remington's Pharmaceutical Sciences (Merck Publishing Co., Easton, Pa.) can be employed. Specific methods for formulation are varied depending on methods for administration. Methods for administration are classified roughly into oral administration and parenteral administration. In the case of the antitumor agent of the present invention, parenteral administration is more preferable.

When the antitumor agent of the present invention is administered parenterally, a specific example thereof is administration by injection. When the antitumor agent of the present invention is administered via injection, the antitumor agent can be prepared as a suspension agent by mixing the recombinant bacterium with a pharmaceutically acceptable solvent, and adding a pharmaceutically acceptable carrier as necessary.

A "pharmaceutically acceptable solvent" may be water or a pharmacologically acceptable aqueous solution (other than water), or an oily fluid. Examples of an aqueous solution include saline and an isotonic solution containing glucose and other auxiliary agents. Examples of auxiliary agents include D-sorbitol, D-mannose, D-mannitol, sodium chloride, as well as low-concentration nonionic surfactants (e.g., polysorbate 80 (TM), HCO-60), and polyoxyethylene sorbitan fatty acid esters. Examples of an oily fluid include sesame oil and soybean oil. The solvent can also be used in combination with benzyl benzoate or benzyl alcohol as a solubilizing agent. Moreover, the solvent may be mixed with a buffering agent such as a phosphate buffer or a sodium acetate buffer, a soothing agent such as procaine hydrochloride, a stabilizer such as benzyl alcohol, or phenol, and an antioxidant.

An injection may be formulated by combining as appropriate and mixing pharmaceutically acceptable excipient, emulsifier, suspension, surfactant, stabilizer, pH adjusting agent, and the like, in a unit dosage form required for generally accepted pharmaceutical practice.

Examples of injection include intravascular injection, intralymphatic injection, intramuscular injection, intraperitoneal injection, and hypodermic injection. Based on the mechanism such that the recombinant bacterium as an active ingredient of the antitumor agent of the present invention grows intratumorally, so as to suppress the proliferation of the tumor cells, when the position of a tumor site is unclear, administration into the cardiovascular system (systemic administration such as intravascular injection or intralymphatic injection) is preferred. Examples of intravascular injection include intravenous injection and intraarterial injection. When the antitumor agent of the present invention is administered, both injections may be applicable. On the other hand, when the position of a tumor site is confirmed, in addition to the above systemic administration, local administration involving direct administration to a tumor may also be employed herein.

The antitumor agent of the present invention can be orally administered according to methods described in the section of Oral Vaccine Agent (described later), thus the explanation therefor is omitted herein.

The content of the recombinant bacterium in the antitumor agent of the present invention may be an amount that allows the cells to reach a target tumor in a state such that they can survive and grow by principally a single administration, and cause no or little harmful side effects on a subject to which the agent is applied. Such contents differ depending on the types of target cells of the antitumor agent, cancer stages, tumor sizes, the number of tumor sites in the whole body, and dosage forms of and administration methods for the antitumor agent, and thus are appropriately determined in view of these conditions.

Objects to which the antitumor agent herein is administered are subjects having tumors or subjects with high possibility of having tumors. The term "subjects" as used herein refers to animals to which the antitumor agent of the present invention is applied. Examples thereof include mammals, preferably humans, dogs, cats, horses, mice, rats, rabbits, cattle, and monkeys, and are preferably humans.

### 2-3. Effects

According to the antitumor agent of the present invention, the recombinant bacterium as an active ingredient survives and grows only within tumors with low oxygen partial pressure and secretes antitumor cell immunotoxin. Therefore, the immunotoxin can be efficiently delivered to tumor cells, and caused to act continuously. Furthermore, when the oxygen partial pressure in the tissue is increased by suppression of tumor cell proliferation and tumor regression due to the effect of immunotoxin, the recombinant bacterium that is an obligate anaerobic gram-positive bacterium becomes unable to survive and thus can be automatically eliminated from the living body. Accordingly, a safe and convenient therapeutic method compared with conventional therapeutic methods using bacteria can be provided, which involves administering intravenously a non-pathogenic obligate anaerobe alone without administering any other antitumor drug, so as to be able to exhibit antitumor effects against the solid cancer.

Furthermore, the antitumor agent of the present invention has an advantage such that it can be administered via intravenous injection and thus has low invasiveness to the subj ect.

### 3. Marker for tumor detection

### 3-1. Overview

A 3^{rd} aspect of the present invention is the recombinant Bifidobacterium for use *in vivo* for tumor detection. The use for tumor detection of the present invention is characterized by using the recombinant bacterium of the 1^{st} aspect as an active ingredient. According to the use for tumor detection of the present invention, the recombinant bacterium as an active ingredient grows only within tumors, and secretes an enzyme, a fluorescent protein, or a luminescent protein within tumors, so that the positions or the sizes of tumors *in vivo* can be monitored.

### 3-2. Configuration

The basic configuration is according to the antitumor agent of the 2^{nd} aspect. Hence, differences with the above antitumor agent are mainly explained herein and the explanation for the configurations that overlap is omitted in principle.

The use for tumor detection of the present invention uses the recombinant bacterium of the 1^{st} aspect as an active ingredient, as described above. The recombinant bacterium in this case is characterized in that a fusion gene encodes a VHH antibody of the family Camelidae recognizing and binding to a surface antigen of target tumor cells, and a functional peptide encodes a labeling protein or a protein inducing labeling. Examples of a labeling protein include the above fluorescent protein and luminescent protein. Examples of a protein inducing labeling include enzymes (luciferase and peroxidase), the substrates of which are luciferin and luminol. Therefore, the recombinant bacterium as an active ingredient of the marker for tumor detection of the present invention secretes extracellular secretory antitumor cell immunomarker (immunolabel).

When the recombinant bacterium of the present invention expresses a fusion gene, its product, an extracellular secretory antitumor cell immunomarker is secreted extracellularly. The secreted immunomarker binds to tumor cells that are target substances with its single-chain antibody moiety, and thus the cells are labeled. Specifically, when a functional peptide is a labeling protein such as a luminescent protein or a fluorescent protein, tumor cells are directly labeled with the labeling protein linked with the single-chain antibody moiety. On the other hand, when a functional peptide is an enzyme, tumor cells are enzymatically labeled with the enzyme linked to the single-chain antibody moiety.

The marker for tumor detection of the present invention can be used in combination with the antitumor agent of the 2^{nd} aspect. In this case, the recombinant bacteria as active ingredients may be the same or different recombinant bacteria that secrete the marker for tumor detection and the antitumor agent separately as individual molecules, and specifically secrete different fusion proteins containing single-chain antibodies that recognize the same target substance. Alternatively, the recombinant bacteria as active ingredients may secrete one fusion protein containing an exotoxin and a labeling protein or an enzyme in its functional protein moiety. In these cases, the same tumor cells can be labeled and also damaged by the effect of the exotoxin.

Moreover, the marker for tumor detection can be used in combination of other antitumor agents, as long as the survival and the growth of the recombinant bacterium as an active ingredient, and the expression and the secretion of the immunomarker are not inhibited or suppressed.

### 3-3. Detection

When the marker for tumor detection of the present invention is administered to a subject, if the subject has a tumor, the recombinant bacterium as an active ingredient grows within the tumor, and an antitumor cell immunomarker is secreted. Tumor cells are labeled with the secreted immunomarker. The labeled tumor cells are detected by luminescence or fluorescence emitted by the labeling protein itself, when the immunomarker is a labeling protein such as a luminescent protein or a fluorescent protein, or alternatively, in the case of enzymatic labeling, detected by luminescence or fluorescence resulting from an enzymatic reaction of a substrate such as luciferin administered *in vivo..* Methods for detecting an immunomarker are not particularly limited. Most tumors are present within a living body. Hence, such a tumor may be exposed by surgery such as laparotomy to detect an immunomarker. An immunomarker can also be detected noninvasively through detection of *in vivo* luminescence or fluorescence from outside the body. Such a noninvasive method that involves detection from outside the body is preferred herein. An example of such a method that can be used herein for detecting luminescence or fluorescence derived from an immunomarker from outside the body is, but is not limited to, an *in vivo* bioimaging method. For example, an immunomarker can be detected, for example, using the methods described in Katz, M.H. et al., 2003, Cancer Res. 63: 5521-5525, Schmitt, C.A. et al., 2002, Cancer Cell, 1: 289-298, Katz, M.H. et al., 2003, J. Surg. Res., 113: 151-160. Such detection can also be performed using a commercially available IVIS Imaging System (Caliper) or an apparatus analogous thereto.

### 3-4. Effects

According to the marker for tumor detection of the present invention, the position and the size of an *in vivo* tumor can be monitored from outside the living body based on an immunomarker. Moreover, a combined use of the marker for tumor detection and the antitumor agent of the 2^{nd} aspect of the present invention enables to suppress tumor cell proliferation due to the immunotoxin, and to detect tumors *in vivo* from outside the living body using the immunomarker, thereby monitoring tumor regression or the therapeutic effects over time.

### 4. Oral vaccine agent

### 4-1. Overview

Also described herein is an oral vaccine agent. The term "oral vaccine agent" refers to a vaccine agent that is orally administered to an individual so as to be able to induce an intestinal immune response in the subject.

The oral vaccine agent is characterized by containing the recombinant bacterium as described herein as an active ingredient. With the use of the oral vaccine agent described herein, the recombinant bacterium as an active ingredient grows within digestive organs and particularly within the intestine and secretes an extracellular secretory antigen peptide (immunoantigen), so that an immune response can be induced against the antigen peptide in an individual, to which the agent has been administered, via the intestinal mucosa.

### 4-2. Configuration

The basic configuration is according to that of the antitumor agent of the 2^{nd} aspect. Hence, differences with the above antitumor agent are mainly explained herein and the explanation for the configurations that overlap is omitted in principle.

The oral vaccine agent contains the recombinant bacterium as described herein as an active ingredient, in a manner similar to that in the antitumor agent of the 2^{nd} aspect. However, the recombinant bacterium in this case differs from that of the antitumor agent of the 2^{nd} aspect in that: a single-chain antibody encoded by a fusion gene recognizes and binds to a surface antigen of intestinal epithelial cells or the like, or mucin or the like that is secreted from goblet cells; and a functional peptide encodes an antigen peptide. The above intestinal epithelial cells may be any of M cells, Paneth cells, goblet cells, absorptive epithelial cells, intestinal endocrine cells, and are preferably M cells existing in follicle-associated epithelium (FAE). Each antigen peptide has 1 or more epitopes as described in the 1^{st} aspect, and various peptides can be used herein for inducing immune responses in individuals to which they are administered.

When the recombinant bacterium expresses a fusion gene in digestive organs, its product, immunoantigen, binds to intestinal epithelial cells or intestinal mucosa, so as to be able to induce an intestinal immune response.

The oral vaccine agent is characterized by inducing an immune response in an individual to which the agent has been orally administered. Therefore, the oral vaccine agent is an orally administered drug.

The oral vaccine agent may be supplemented with, in addition to the recombinant bacterium as an active ingredient, a pharmaceutically acceptable carrier.

The term "pharmaceutically acceptable carrier" refers to a substance that facilitates the formulation of a drug or the application to a living body, and is added for maintaining the survival of the recombinant bacterium as an active ingredient, as long as it does not inhibit or suppress the effect of the cells. Examples thereof include an excipient, a binder, a disintegrator, a filler, an emulsifier, a fluid modulator to be added, and a lubricant.

Examples of an "excipient" include sugars such as monosaccharides, disaccharides, cyclodextrins and polysaccharides (specific examples thereof include, but are not limited to, glucose, sucrose, lactose, raffinose, mannitol, sorbitol, inositol, dextrin, maltodextrin, starch and cellulose), metallic salts (e.g., sodium phosphate or calcium phosphate, calcium sulfate, and magnesium sulfate), citric acid, tartaric acid, glycine, low-, medium-, and high-molecular-weight polyethylene glycols (PEG), pluronic, and combinations thereof.

Examples of a "binder" include the paste of the starch of corn, wheat, rice, or potato, gelatin, tragacanth, methylcellulose, hydroxypropyl methylcellulose, sodium carboxymethylcellulose and/or polyvinylpyrrolidone.

Examples of a "disintegrator" include the aforementioned starchs, carboxymethyl starch, crosslinking polyvinylpyrrolidone, agar, alginic acid or sodium alginate, and salts thereof.

Examples of a "filler" include the aforementioned sugars and/or calcium phosphate (e.g., tricalcium phosphate or dibasic calcium phosphate).

Examples of an "emulsifier" include a sorbitan fatty acid ester, a glycerin fatty acid ester, a sucrose fatty acid ester, and a propylene glycol fatty acid ester.

Examples of a "fluid modulator to be added" and a "lubricant" include, silicate, talc, stearate and polyethylene glycol.

In addition to the above examples, if necessary, a taste and flavor corrigent, a suspension, a diluent, a surfactant, an extending agent, a humidifying agent, a moisturizing agent (e.g., glycerin and starch), an adsorbent (e.g., starch, lactose, kaolin, bentonite, and colloidal silicic acid), a disintegration-suppressing agent (e.g., saccharose, stearin, cocoa butter, and hydrogenated oil), a coating agent, a colorant, a preservative, an antioxidant, aroma chemicals, a flavoring agent, a sweetening agent, and a buffering agent. One, two or more carriers above may be adequately used as necessary.

Examples of the dosage form of an oral vaccine agent include solids (e.g., tablets, pills, sublingual agents, capsules, and drops), granules, dust formulations, powders, and solutions. Furthermore, solids can be formulated as necessary into dosage forms via coating known in the art, such as sugar-coated tablets, encapsulated gelatin tablets, enteric-coated tablets, film-coated tablets, double layer tablets, and multilayer tablets. Specific shapes and sizes of such dosage forms are not particularly limited, as long as each of all the dosage forms is within the range of the dosage forms known in the art.

### 4-3. Effects

The oral vaccine agent can be orally administered noninvasively, unlike conventional vaccine agents that are administered to a living body via injection or the like.

The use of the oral vaccine agent makes it possible to continuously provide an immunoantigen as a vaccine to an individual since the recombinant bacterium as an active ingredient can survive and grow persistently within the intestine.

### <Example 1: Configuration of expression cassette>

The structure of and a method for constructing an expression cassette for introduction of *Bifidobacterium* to be used in the following examples as obligate anaerobic gram-positive bacteria are as described below.

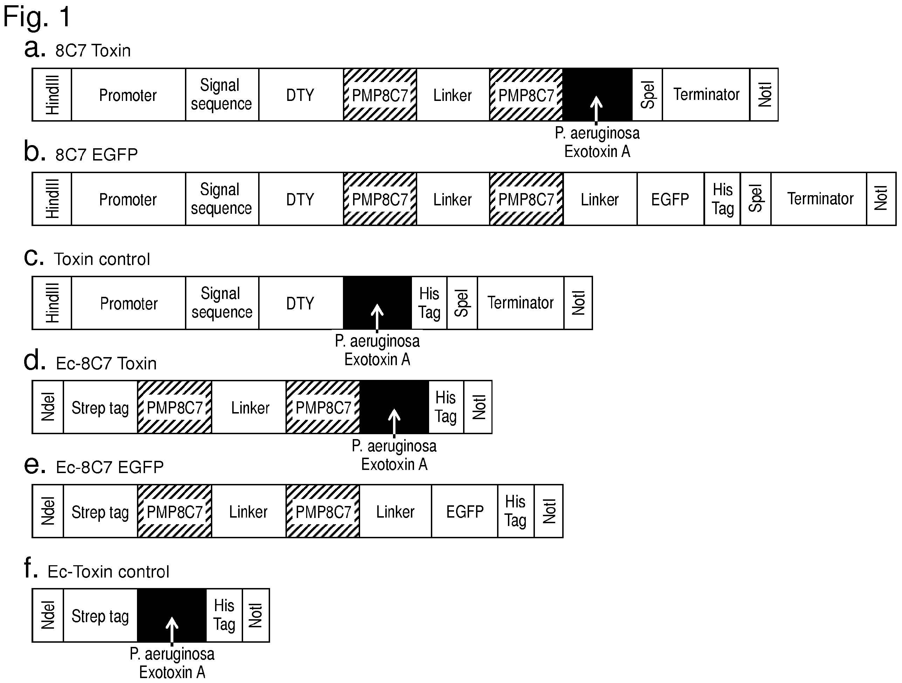

### (1) Expression cassette of anti-EGFR antibody/Pseudomonas aeruginosa exotoxin A fusion protein (hereinafter, referred to as "8C7 toxin") (Fig. 1a; SEQ ID NO: 1/amino acid sequence: SEQ ID NO: 16)

The fusion protein expression cassette contains a fusion gene having the structure described in the 1^{st} aspect of the present invention. The expression cassette was constructed by designing a fusion gene, in which a *Hind* III cleavage site, promoter region (SEQ ID NO: 4) of hup gene derived from the *B. longum,* usp secretory signal sequence (SEQ ID NO: 5) derived from the *B. longum,* DTY (insertion sequence following signal sequence), a coding sequence of two lama anti-EGFR single-chain antibody (PMP8C7 ; SEQ ID NOS: 7 and 8; JP Patent Publication (Kokai) No. 2008-534933, JP Patent Publication (Kohyo) No. 2009-511032) linked with a sequence (SEQ ID NO: 6) encoding the linker peptide (GGSGG)₂, a coding sequence of the *Pseudomonas aeruginosa* exotoxin A prepared by removing a domain involved in cell adhesion as an exotoxin (SEQ ID NO: 9 ; Brinkmann U., et al., 1994, Biochimica et Biophysica Acta, 1198: 27-45), a termination codon, an *Spe* I site, terminator of the histone-like protein (HUT) (SEQ ID NO: 10), and a *Not* I cleavage site were linked in this order, and then preparing by DNA synthesis. Subsequently, the resultant was cloned into a pBluescript plasmid (life technologies), thereby confirming the DNA sequence.

### (2) Expression cassette of an anti-EGFR antibody/EGFP fusion protein (hereinafter, referred to as "8C7 EGFP") (Fig. 1b; SEQ ID NO: 2)

The expression cassette is an expression cassette as a control or for detecting the localization, which is prepared by linking a fluorescent protein EGFP instead of an exotoxin. The expression cassette was prepared by designing a gene having the same structure ranging from the *Hind* III cleavage site to two alpaca anti-EGFR single-chain antibodies as that of the above 8C7 toxin, followed by a linker peptide (GGSGG)₂-encoding sequence, EGFP (Zhang G., et al., 1996, Biochem Biophys Res Commun, 227 (3): 707-11), a termination codon, an *Spe* I cleavage site, HUT, a His-Tag sequence, and a *Not* I cleavage site linked in this order, and then preparing by DNA synthesis. Subsequently, the resultant was cloned into a pBluescript plasmid, thereby confirming the DNA sequence.

### (3) Expression cassette of Pseudomonas aeruginosa exotoxin A protein (hereinafter, referred to as "Toxin control") (Fig. 1c; SEQ ID NO: 3)

This expression cassette is a control expression cassette consisting of only an exotoxin containing no single-chain antibody moiety. The expression cassette had a structure in which a His-Tag sequence had been added to the C-terminus of the coding the sequence of *Pseudomonas aeruginosa* exotoxin A prepared by removing two lama anti-EGFR single-chain antibody-encoding sequences including a linker peptide from 8C7 toxin, so as to remove a cell adhesion domain (SEQ ID NO: 9), and was prepared by DNA synthesis. Subsequently, the resultant was cloned into a pBluescript plasmid, thereby confirming the DNA sequence.

### <Example 2: Cell proliferation-inhibiting activity of fusion protein 8C7 toxin>

In this example, the preparation of recombinant *E. coli* BL21 (DE3) expressing fusion proteins such as 8C7 toxin as described in Example 1 and the cell proliferation-inhibiting activity of the fusion proteins were verified.

### (1) Construction of Ec-8C7 toxin, Ec-Toxin control, and Ec-8C7 EGFP expression vectors for expression in Escherichia coli

The Ec-8C7 toxin expression cassette for expression in *Escherichia coli* was amplified by PCR using 8C7 toxin inserted into a pBluescript plasmid as a template and a primer set of SEQ ID NOS: 11 and 12. PCR was performed using PrimeSTAR GXL DNA Polymerase (Takara Bio Inc., Otsu-shi, Japan) as DNA polymerase under conditions of 1 cycle of 95°C for 1 minute, and 25 cycles of 98°C for 10 seconds, 60°C for 15 seconds, and 68°C for 2 minutes. The thus amplified product was purified using a MinElute column (Qiagen) according to the protocols included therewith, digested with restriction enzymes *Nde* I and *Not* I, and then subjected to electrophoresis with 1.2% agarose. A target band was excised from the gel and then purified and isolated using a DNA gel extraction kit (Qiagen) according to the protocols included therewith. The resultant was inserted into a restriction site corresponding to the restriction sites in the multi-cloning site of pET-22b (+) (Novagen). Thus, Ec-8C7 toxin containing Strep Tag (Schmidt T.G., Skerra A., 2007, Nat Protoc. 2 (6): 1528-35), two PMP8C7 sequences linked with a (GGSGG)₂ linker peptide, the sequence of *Pseudomonas aeruginosa* exotoxin A from which a domain involved in cell adhesion had been removed, and His Tag was constructed (Fig. Id).

Ec-8C7 EGFP expression cassette for expression in *Escherichia coli* was amplified by PCR using as a template the expression cassette of anti-EGFR antibody/EGFP fusion protein, which is inserted into a pBluescript plasmid and a primer set of SEQ ID NOS: 13 and 14. PCR conditions were the same as those in the above. The thus amplified product was purified using a MinElute column according to the protocols included therewith. After digestion with restriction enzymes *Nde* I and *Not I,* electrophoresis was performed with 1.2% agarose, a target band was excised from the gel and then purified and isolated using a DNA gel extraction kit according to the protocols included therewith. The resultant was inserted into a restriction site corresponding to the MCS of pET-22b (+). Thus, Ec-8C7 EGFP containing Strep Tag, two PMP8C7 sequences linked with a (GGSGG)₂ linker peptide, a green fluorescent protein (EGFP), and His Tag was constructed (Fig. 1e).

The Ec-Toxin Control expression cassette for expression in *Escherichia coli* was amplified by PCR using as a template a Toxin control expression cassette inserted into a pBluescript plasmid and a primer set of SEQ ID NOS: 15 and 12. PCR conditions were the same as those described above. The thus amplified product was purified using a MinElute column according to the protocols included therewith. After digestion with restriction enzymes *Nde* I and *Not* I, and then electrophoresis was performed with 1.5% agarose. A target band was excised from the gel and then purified and isolated using a DNA gel extraction kit according to the protocols included therewith. The resultant was inserted into a restriction site corresponding to the MCS of pET-22b (+). Thus, an Ec-Toxin Control containing Strep Tag, the sequence of *Pseudomonas aeruginosa* exotoxin A from which a domain involved in cell adhesion had been removed, and His Tag was constructed (Fig. If).

### (2) Expression and purification of Ec-8C7 toxin, Ec-Toxin control, and Ec-8C7 EGFP

pET-22b (+) plasmid DNA containing each expression cassette constructed in (1) above was introduced into *E. coli* BL21 (DE3) competent cells (Novagen) or BL21Star™ (DE3) One Shot competent cells (Invitrogen) according to the protocols included therewith. The recombinant protein was expressed in each transformant under the following conditions. *E.coli* BL21 (DE3) transformant clone was cultured in a 2YTA medium (2YT medium containing 100 µg/mL ampicillin) at 37°C. When OD reached OD600 = 0.5 to 0.6, IPTG (Isopropyl-β-D-thiogalactopyranoside, Takara Bio Inc.) was added to the medium to a final concentration of 1 mM (0.5 mM in the case of BL21 (DE3) StarTM strain), and then cells were cultured at 30°C for 3 hours. After collected, cells were suspended again in 10 mL of an extraction buffer (50 mM Na Phosphate pH7.8, 300 mM NaCl, EDTA-Free protease inhibitor cockatail (Roche)) per 100 mL of the culture solution. Ultrasonication was performed in ice using Sonifier 250 (Branson) under conditions of Output Control 2 and Duty cycle 50%, thereby disrupting the cells. After disruption, the suspension was centrifuged at 15000 rpm and 4°C for 15 minutes, thereby collecting the supernatant. The fusion protein was purified using a HisTrap column (GE Healthcare UK, Buckinghamshire, England). The thus centrifuged supernatant of the ultrasonicated suspension was directly applied to a HisTrap column, the column was washed with a binding buffer (20 mM Na Phosphate, 0.5M NaCl, 20 mM Imidazole, pH7.4), and then the fusion protein was eluted with a solution for elution containing 500 mM Imidazole.

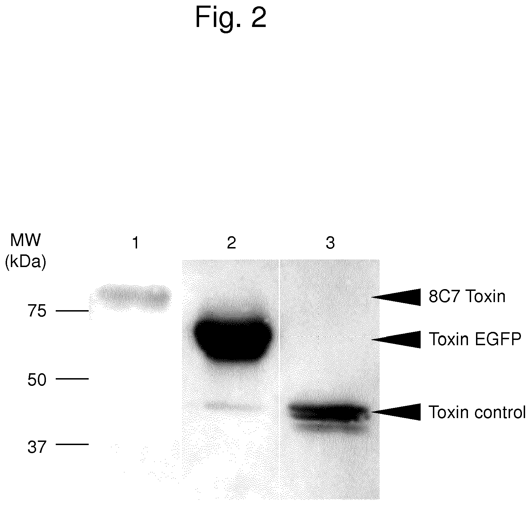

The results of purification were detected and confirmed by Western blotting using an anti-His Tag antibody against a His tag positioned at the C-termini of all fusion proteins. As a result, bands were each detected at positions as predicted (data not shown). Moreover, each fusion protein was confirmed to have a full length using StrepTactin HRP (Bio-rad) that binds to Strep Tag on the N-terminus of the recombinant protein. Specifically, it was detected by washing the membrane, adding a chemiluminescence reagent LuminataTM Forte, and then using ImageQuantLAS4000 (Fujifilm/GE). The result is shown in Fig 2. Purification of full-length Ec-8C7 toxin, Ec-8C7 EGFP, and Ec-Toxin control was confirmed from these results. The thus obtained fusion proteins were preserved at 4°C after addition of 10% glycerin.

### (3) Cell proliferation-inhibiting activity of Ec-8C7 toxin

The proliferation-suppressing effects of each fusion protein on A431 cells line overexpressing EGFR (cells derived from human skin cancer, which were obtained from the RIKEN BioResource Center, Tsukuba-shi, Japan), BxPC-3 cells line expressing EGFR at low expression levels (cells derived from human pancreatic cancer, which were obtained from ATCC, Manassas, U.S.A.) (Folia Pharmacol. Jpn. 2011, 137; 31-41), and HT-29 cells (i.e., cells derived from human colon cancer, which were obtained from ATCC, Manassas, U.S.A.) were examined. Cells were added to a Falcon 96-well culture plate (Becton, Dickinson and Company) at 2.5 × 10⁴ cells/50 µL medium (DMEM containing 0.2% fetal calf serum (Tissue Culture Biologicals, Tulare, U.S.A.)/well. After 3 hours of culture, 50 µL each of media containing Ec-8C7 toxin, Ec-8C7 EGFP, and Ec-Toxin control (purified in (2) above) at 400, 200, 100, 50, and 25 ng/mL, was added. The aforementioned three types of cell line do not die, but almost never proliferate under conditions of DMEM containing 0.2% fetal calf serum and 3 days of culture. Regarding Ec-8C7 toxin, Ec-8C7 EGFP, and Ec-Toxin control, samples purified using a HisTrap column (GE Healthcare NJ, U.S.A.) were used. Hence, in view of the buffer components to be mixed upon addition to media, a buffer control was used. On day 3 of the culture, 10 µL of a MTT reagent (Nacalai Tesque, Inc.) was added. Moreover, after 2 hours of culture, a lysate was added at 100 µL/well, cells were lysed by pipetting, and then absorbance was measured at OD₅₇₀ nm. The measured value of a cell-free medium in a well was used as a background value and was subtracted from all the measured values. The results are indicated relative to the result for a well containing a buffer control at each dilution level, which was designated as 100%. Each assay was performed in duplicate, and an average value of the results for 2 wells was used.

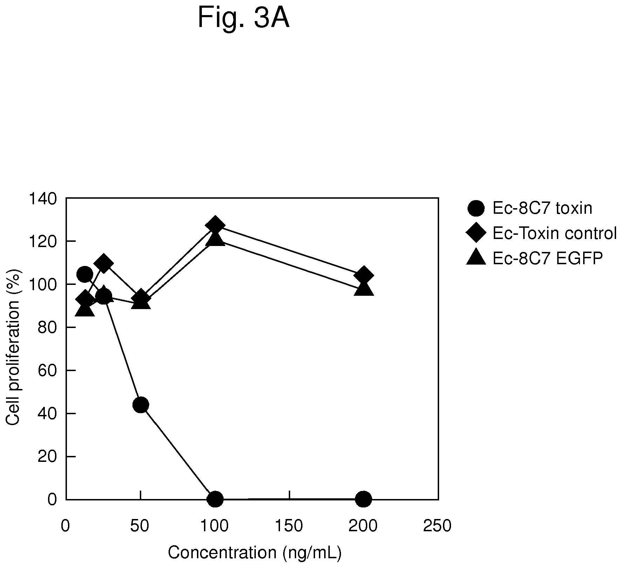

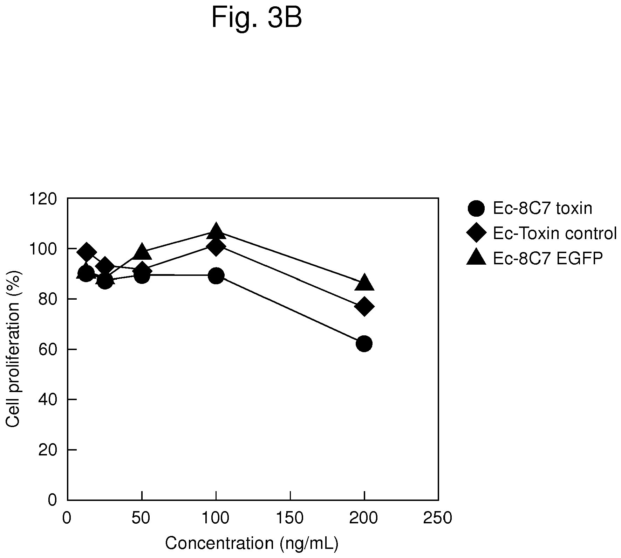

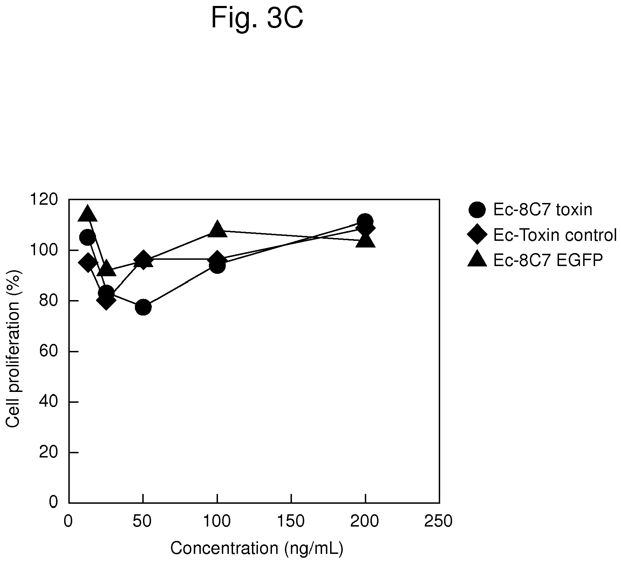

The results are shown in Fig. 3. Fig. 3a shows the results of A431 cells, Fig. 3b shows the results of BxPC-3 cells, and Fig. 3c shows the results of HT-29 cells. As shown in Fig. 3a, regarding A431 cells, Ec-8C7 toxin shows concentration-dependent activity to inhibit the proliferation, and IC₅₀ was 0.68 nmol/L (47.5 ng/mL). In contrast, regarding the negative control, Ec-Toxin control, no activity to inhibit proliferation was detected. Therefore, Ec-8C7 toxin was demonstrated to have target-cell-specific cell proliferation-inhibiting activity. The results suggest that Ec-8C7 toxin was bound to EGFR via anti-EGFR antibody moiety, introduced into the cells by EGFR internalization (McClintock J.L. & Ceresa B.P., 2010, Invest Ophthalmol Vis Sci. 51 (7) 3455-3461), and then the exotoxin moiety was translocated into the cytoplasm, EF-2 was inactivated by ADP ribosylation thereof, and thus the protein synthesis by A431 cells was inhibited. Furthermore, the result that no cell proliferation-inhibiting activity was detected for the negative control Ec-8C7 EGFP suggested that even if Ec-8C7 EGFP is bound to EGFR via anti-EGFR antibody moiety, it cannot inhibit the signal transduction of EGFR because of the lack of the exotoxin moiety, having no effects on cell proliferation. In contrast, as shown in Fig. 3b, regarding BxPC-3 cells expressing EGFR at low levels, Ec-8C7 toxin was detected to have weak proliferation-inhibiting activity (about 40% inhibitory activity in the case of 200 ng/mL 8C7 toxin). Moreover, as shown in Fig. 3c, the activity of Ec-8C7 toxin to inhibit the proliferation of HT-29 cells was not detected.

### <Example 3: The binding of 8C7 EGFP to tumor cells and measurement of the dissociation constant of 8C7 EGFP and EGFR ECD>

In this example, the binding of Ec-8C7 EGFP described in Example 2 to living cells expressing EGFR was examined.

### (1) Binding of Ec-8C7 EGFP to tumor cells

A431 cell line overexpressing EGFR and an HT-29 cell line expressing EGFR at low levels were separately plated on Falcon 24-well plates (Becton, Dickinson and Company) at 2.5 × 10⁵ cells/0.5 mL medium (DMEM containing 0.2% fetal calf serum)/well. On the next day, each medium was exchanged with DMEM containing 0.2% fetal calf serum containing Ec-8C7 EGFP purified in Example 2 at 10 µg/mL. After 2 hours of culture, the above media were removed, and then washed twice with PBS (Mg²⁺ and Ca²⁺ free-phosphate-buffered saline). PBS was added at 0.5 mL/well, and then the cells were observed with a fluorescence microscope (ECLIPS Ti, Nikon). Because of the use of Ec-8C7 EGFP purified using a HisTrap column, a buffer control was used in view of a buffer component to be mixed upon addition to each medium.

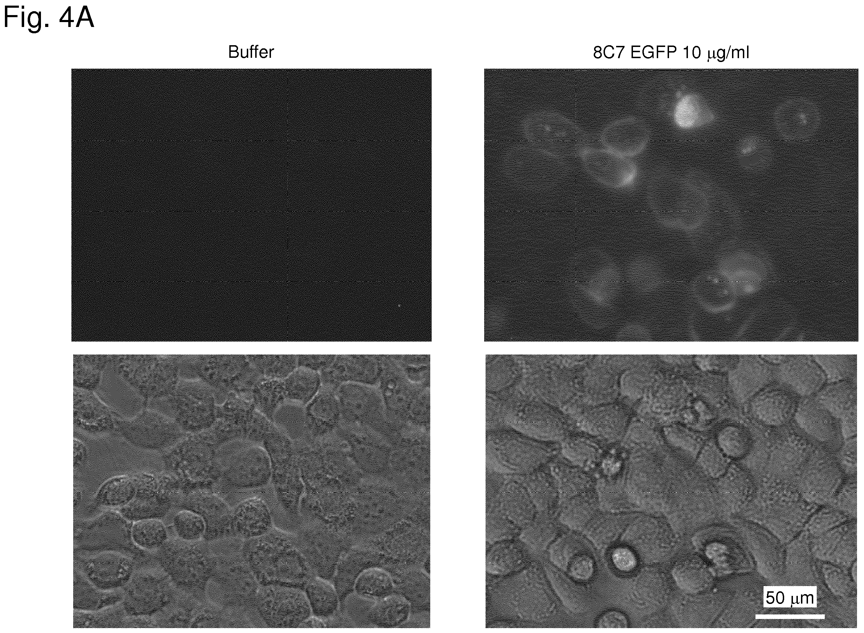

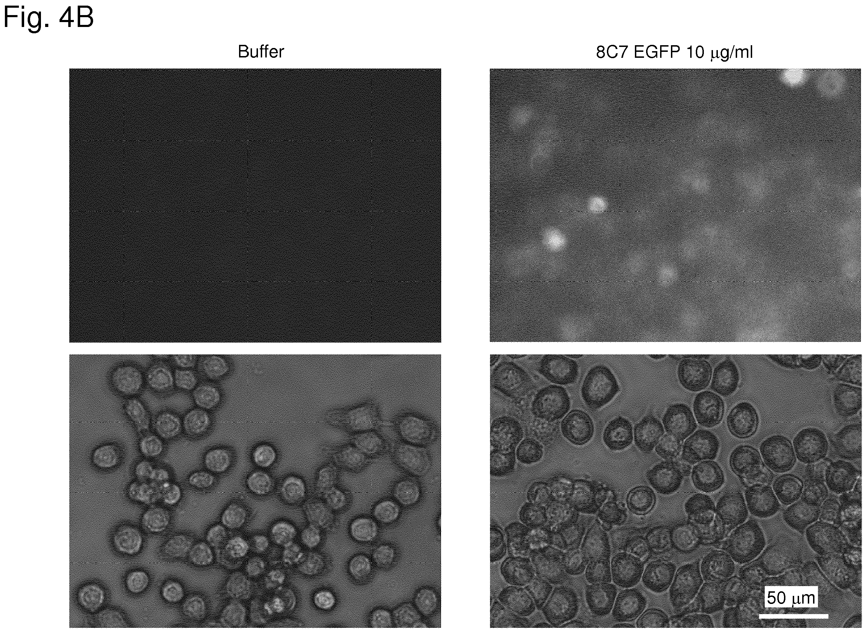

The results are shown in Fig. 4a and Fig. 4b. In Fig. 4a, Ec-8C7 EGFP fluorescence was detected on the surface of A431 cells, and thus the binding of Ec-8C7 EGFP to A431 cells was successfully detected. Also, EGFR internalization was observed in some cells. In contrast, with regard to HT-29 cells in Fig. 4b, no Ec-8C7 EGFP fluorescence was detected for, revealing that Ec-8C7 EGFP does not bind to HT-29 cells.

### (2) Detection of EGFR internalization with Ec-8C7 EGFP

EGFR internalization was observed over time using A431 cells. A431 cells were seeded on 3 dishes containing 1 × 10⁶ cells/2 mL medium (DMEM containing 10% fetal calf serum)/35 mmcp dish. After 1 day of culture, each medium was exchanged with a medium containing 10 µg/mL Ec-8C7 EGFP (DMEM containing 10% fetal calf serum). One dish was subjected to 1 hour of incubation at 4°C, one dish was subjected to 2 hours of incubation at 37°C, and the remaining one dish was subjected to 4 hours of incubation at 37°C. Dishes were washed twice with 2 mL of PBS/dish, and then observed with a fluorescence microscope.

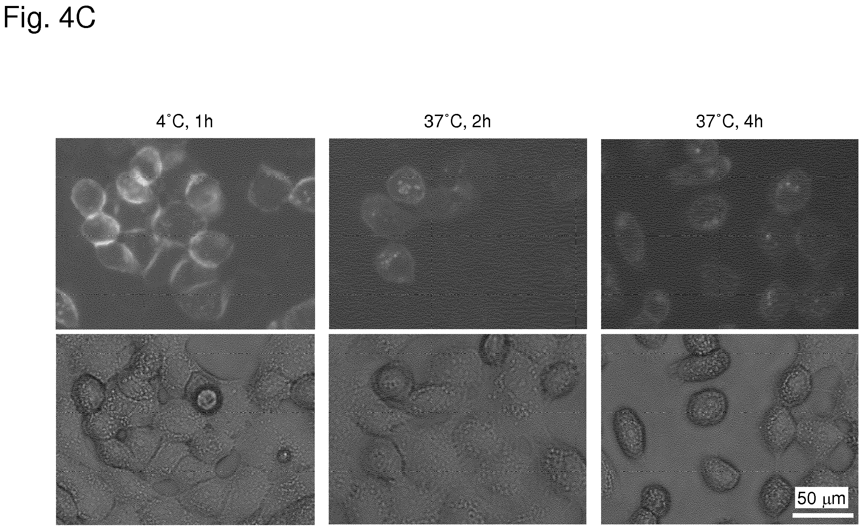

The results are shown in Fig. 4C. Under conditions of 37°C, EGFR internalization was successfully observed in most cells.

### (3) Measurement of dissociation constant (K_{D}) of Ec-8C7 EGFP and EGFR extracellular domain (ECD; extracellular domain)

Binding affinity between Ec-8C7 EGFP and recombinant human EGFR ECD (Sino Biological, Beijing, China) was analyzed by a surface plasmon resonance method using Biacore 3000 (GE Healthcare, NJ, U.S.A.). Measurement was performed by a single cycle kinetics method (Analytical Biochemistry Volume 349, Issue 1, 1 February 2006, Pages 136-147). Ec-8C7 EGFP was added sequentially at 1.56 nM (1^{st}), 3.13 nM (2^{nd}), 6.25 nM (3^{rd}), 12.5 nM (4^{th}), and then at 25 nM (5^{th}) and then measured.

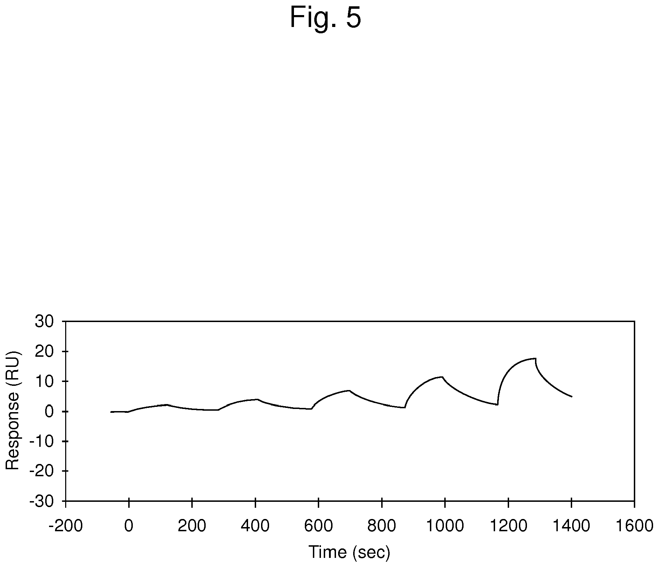

The results are shown in Fig. 5. The K_{D} value of Ec-8C7 EGFP and recombinant human EGFR ECD was found to be 11 nM

### <Example 4: Accumulation of recombinant Bifidobacterium in tumors and its effects on mice>

Recombinant *Bifidobacterium* expressing and secreting 8C7 toxin was intravenously administered to a Xenograft model prepared to form solid cancer by transplanting A431 cells to the nude mouse back. The accumulation of the recombinant *Bifidobacterium* in tumors, and the effects on mice, were verified.

### (1) Construction of vector for the expression of 8C7 toxin and 8C7 toxin-expressing recombinant Bifidobacterium

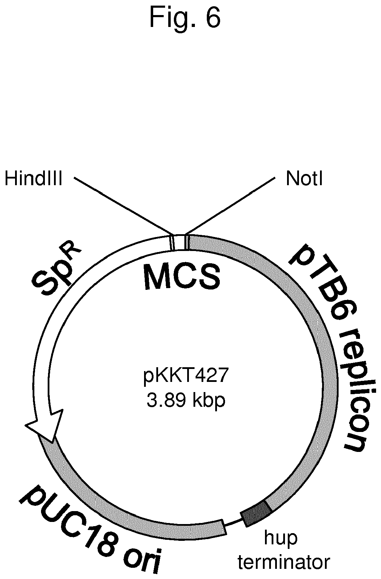

As a vector, a shuttle vector pKKT427 (Fig. 6; Yasui K, et al., 2009, Nucleic Acids Res., 37 (1):e3) of *Bifidobacterium* and *Escherichia coli* containing replicon of pTB6 (Tanaka K, et al., 2005, Biosci Biotechnol Biochem. 69 (2): 422-425) was used. An expression cassette for *Bifidobacterium* (Fig. 1a) was inserted between *Hind* III and *Not* I at the MCS (multicloning site) of pKKT427. The thus constructed expression vector was introduced into *Bifidobacterium B. longum* 105-A (provided by the former professor of the Kyoto Pharmaceutical University, Yasumasa Kano) using an electroporation method. Electroporation was performed under conditions of 2.4 kV/cm, 25 µF, and 200Ω. The thus obtained recombinant *Bifidobacterium* was suspended in saline again, thereby preparing a solution to be administered to nude mice.

### (2) Accumulation of recombinant Bifidobacterium in tumors

2 × 10⁶ cells of A431 were subcutaneously transplanted into each 6-week-old female KSN/Slc nude mouse (Japan SLC, Inc., Hamamatsu, Japan). After 2 weeks from the transplantation of solid cancer, 1.5 × 10⁹ cells of the recombinant *Bifidobacterium* prepared in (1) above were intravenously administered. As a negative control, a nude mouse that had intravenously received no recombinant *Bifidobacterium,* was used. As a nutritional supplement for *in vivo* recombinant *Bifidobacterium,* 1 mL of 20% lactulose (Wako Pure Chemical Industries, Ltd., Osaka, Japan) was intraperitoneally administered daily to all mice. One week after administration, tumors (n=5, average value of 277.2 mm³) were collected from mice, and blood and spleens were also collected from mice as control. Blood was directly spread, and tumors and spleens homogenized in MRS medium (Lactobacilli MRS Broth (Difco Laboratories, Detroit, MI), 50 mM sucrose, 3.4 mg/mL L-ascorbic acid sodium salts, and 0.2 mg/mL L-cysteine hydrochloride) was spread, on each MRS agar medium containing 100 µg/mL spectinomycin, which was prepared by adding 1.5% Bacto Agar (Difco Laboratories) to the above MRS liquid medium. After 2 days of culture at 37°C under an anaerobic environment prepared by adding Anaero Pack Kenki (Mitsubishi Gas Chemical Company, Inc., Tokyo, Japan), which was a deoxidizer, into a container or a bag to create an hypoxic condition allowing *Bifidobacterium* to grow and then colonies that had appeared on the MRS selective agar media were measured.

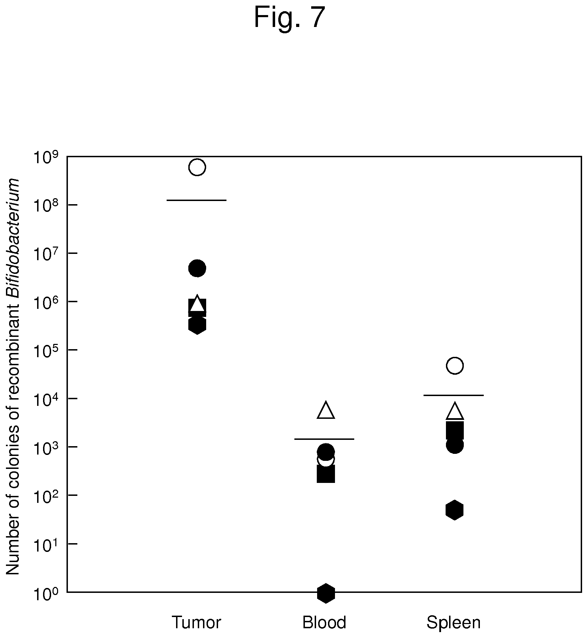

The results are shown in Figure 7. As shown in Figure 7, the recombinant *Bifidobacterium* was revealed to have accumulated in tumors with selectivity 100 to 1000 times greater than that in blood or spleen.

### (3) Changes in body weight and GPT level by intravenously administering recombinant Bifidobacterium

The body weights of nude mice treated with the recombinant *Bifidobacterium* described in (2) were measured on Day 0, on which the recombinant *Bifidobacterium* was intravenously administered, and Week 1 (Day 7 after administration), on which tumors were collected. As a negative control, a mouse to which A431 cells had been transplanted but no recombinant *Bifidobacterium* had been intravenously administered was used. Measurement was performed for each group with n = 5.

The results are shown in Table 1.

**Table 1**

| Recombinant *Bifidobacterium* | Day 0 | Day 7 |
|---|---|---|
| Not administered | 1.00±0.03 | 1.00±0.03 |
| Administered | 1.00±0.03 | 1.08±0.04 |

As shown in Table 1, no body weight loss was detected in mice due to intravenous administration of the recombinant *Bifidobacterium.*

Serum was prepared from blood collected from each of nude mice treated with the recombinant *Bifidobacterium* of (2) above and a negative control mouse. GPT (ALT) levels were measured using Transamylase CII·Test Wako (Wako Pure Chemical Industries, Ltd.) to examine the presence or the absence of hepatic dysfunction. Serum was prepared from the collected blood at room temperature for 30 minutes and then overnight at 4°C, performing centrifugation at 1,000 × g, and then collecting the supernatant.

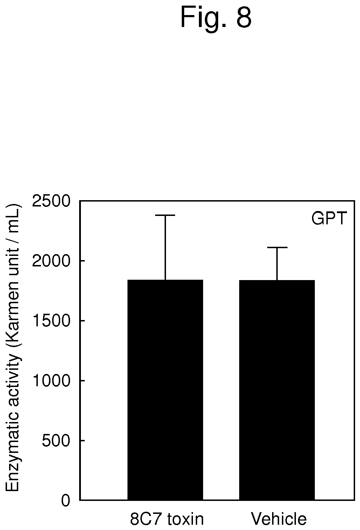

The results are shown in Fig. 8. No significant changes were observed in GPT level for the group to which the recombinant *Bifidobacterium* had been intravenously administered (8C7 toxin), compared with the negative control (Vehicle).

The above results suggest that even if the recombinant *Bifidobacterium* secreting a fusion protein 8C7 toxin that contains an exotoxin having cytotoxic activity is intravenously administered to mice, this causes almost no side effects in the living bodies.

### <Example 5: In vivo detection of EGFR internalization using recombinant Bifidobacterium expressing 8C7 EGFP>

### (1) Construction of 8C7 EGFP expression vector and preparation of recombinant Bifidobacterium

A 8C7 EGFP expression vector was constructed by inserting an expression cassette 8C7 EGFP for *Bifidobacterium* (Fig. 1b) between *Hind* III and *Not* I at the MCS of the shuttle vector pKKT427 described in Example 4. The thus constructed 8C7 EGFP expression vector was introduced into *B. longum* 105-A using an electroporation method. Electroporation was performed under the conditions of 2.4 kV/cm, 25 µF, and 200Ω. The thus obtained recombinant *Bifidobacterium* was suspended in saline again, thereby preparing a solution to be administered to nude mice.

### (2) Administration of recombinant Bifidobacterium to cancer-bearing mice prepared using A431 cells and detection of EGFR internalization

2 × 10⁶ cells of A431 were subcutaneously transplanted into each 6-week-old female KSN/Slc nude mouse. After two weeks from the formation of solid cancer, 1.5 × 10⁹ cells of the recombinant *Bifidobacterium* prepared in (1) above were intravenously administered. As a negative control, a nude mouse that had intravenously received no recombinant *Bifidobacterium,* was used. As a nutritional supplement for *in vivo* recombinant *Bifidobacterium,* 1 mL of 20% lactulose (Wako Pure Chemical Industries, Ltd., Osaka, Japan) was intraperitoneally administered daily to all mice. One week after administration, tumors (n=10, average volume of 450 mm³) were collected from mice and then cryopreserved in an embedding agent for preparation of frozen tissue sections, Tissu-Tek O.C.T. Compound (Sakura Finetek Japan, Tokyo, Japan). Frozen sections were prepared to have a thickness of 10 µm using a freezing microtome, Leica CM3050 S (Leica, Wetzlar, Germany). Prolong Antifade Reagent with DAPI (Life Technologies, Carlsbad, CA) was used as a mounting medium and nuclear staining was performed with DAPI (4',6-Diamidino-2-phenylindole) upon mounting. Micro Slide Glass (Matsunami Glass Ind.,Ltd., Osaka, Japan) was used as slide glass and Neo Micro Cover Glass (Matsunami Glass Ind.,Ltd.) was used as cover glass. Eclipse 80i (Nikon, Tokyo, Japan) was used as a fluorescence microscope. Cell nuclei stained with DAPI were detected as fluorescence at around 400 nm using exciting light with a wavelength of 340-380 nm. 8C7 EGFP was detected as fluorescence at around 505 nm using exciting light with a wavelength of 465-495 nm.

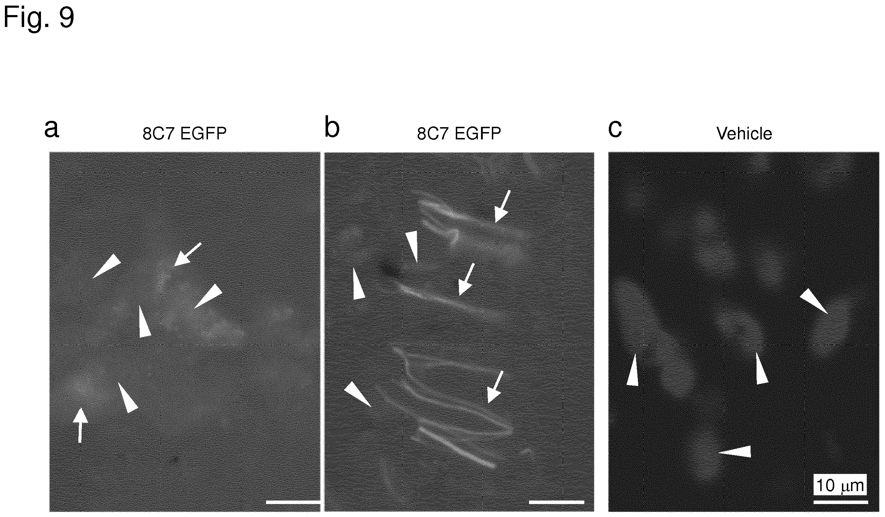

The results are shown in Fig. 9. Fig. 9a and Fig. 9b show 8C7 EGFP and Fig. 9c shows the negative control. In these figures, 8C7 EGFP fluorescence is indicated with arrows and DAPI fluorescence is indicated with arrow heads. Green EGFP fluorescence was observed in the form of dots around A431 cell nuclei, revealing that the EGFR internalization of 8C7 EGFP also took place *in vivo.* This suggests that when the recombinant *Bifidobacterium* expressing and secreting 8C7 toxin is administered intravenously, *Pseudomonas aeruginosa* exotoxin A is incorporated into cancer cells and exhibits its strong cytotoxic effect against cancer cells, even *in vivo.* Also, as shown in Fg. 9b, linear fluorescence images (arrows) were observed. This suggests that the recombinant *Bifidobacterium* linearly inhabits (Yazawa K, et al., 2000, Cancer Gene Therapy 7: 269-274). Similar results were also obtained for human-pancreatic-cancer-derived BxPC-3 cells (data not shown).

### <Example 6: Antitumor effect in the case of intravenous administration of 8C7 toxin-expressing recombinant Bifidobacterium (1)>

Antitumor effect in the case of intravenous administration of 8C7-toxin-expressing recombinant *Bifidobacterium* into Xenograft model of a A431 cell was verified.

### (1) Construction of vector for the expression of toxin control as negative control and preparation of recombinant Bifidobacterium

As a negative control for examining antibody-dependent antitumor effects, the Toxin control described in Fig. 1c was used. Toxin control which is the expression cassette for *Bifidobacterium* (Fig. 1c) was inserted between *Hind* III and *Not* I at the MCS of the shuttle vector pKKT427 described in Example 4. The Toxin control expression vector thus constructed was introduced into *B. longum* 105-A using an electroporation method. Electroporation was performed under the conditions of 2.4 kV/cm, 25 µF, and 200Ω. The thus obtained recombinant *Bifidobacterium* was suspended in saline again, thereby preparing a solution to be administered to nude mice.

### (2) Measurement of the antitumor effects of recombinant Bifidobacterium in Xenograft model of A431 cell

2 × 10⁶ cells of A431 were subcutaneously transplanted into each 6-week-old female KSN/Slc nude mouse. After 8 days from transplantation, mice were grouped so that each group (Vehicle, 8C7 toxin, Toxin control; n = 5 each) had about an average tumor volume of 130 mm³. The 8C7-toxin-expressing recombinant *Bifidobacterium* prepared in Example 4 (1) and the Toxin-control-expressing recombinant *Bifidobacterium* prepared in Example 6 (1) were intravenously administered (1.5 × 10⁹ cells each). As a negative control (Vehicle), a nude mouse that had intravenously received no recombinant *Bifidobacterium,* was used. As a nutritional supplement for *in vivo* recombinant *Bifidobacterium,* 1 mL of 20% lactulose (Wako Pure Chemical Industries, Ltd., Osaka, Japan) was intraperitoneally administered daily to all mice. Tumor size was measured using a vernier caliper on days 8, 10, 15, 18, and 20 after subcutaneous transplantation of A431 cells. Tumor volume was calculated via the equation of "(shorter diameter)² × (longer diameter)/2".

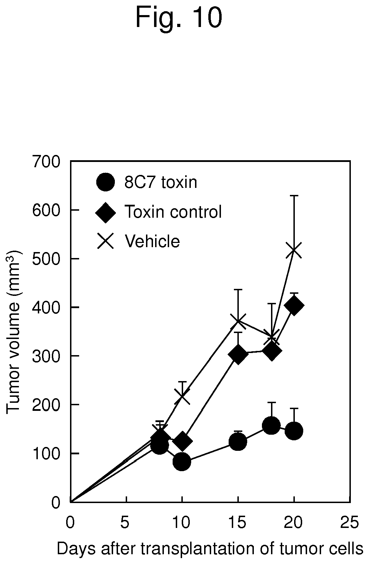

Results are shown in Fig. 10. 8C7-toxin-expressing recombinant *Bifidobacterium* was observed to exhibit the effect of suppressing tumor growth by 93%, compared with negative control (Vehicle). In contrast, almost no effect of suppressing tumor growth was observed for the recombinant *Bifidobacterium* expressing Toxin control as a negative control, containing no anti-EGFR single-chain antibody PMP8C7. These results suggest that, *in vivo,* the 8C7 toxin secreted from the recombinant *Bifidobacterium* was bound to the EGFR receptor on the surface of A431 cells via the anti-EGFR single-chain antibody moiety, and thus a cytotoxic effect was exhibited because of the *Pseudomonas aeruginosa* exotoxin A moiety. The antitumor effect obtained herein is equivalent to the effect of suppressing tumor growth of gefitinib that is a low-molecular-weight molecular target drug selectively inhibiting EGFR tyrosine kinase (James G., et al., 2001, Clin Cancer Res, 7:4230-4238) and thus is revealed to be sufficient as the effect of suppressing tumor growth.

### <Example 7: Antitumor effect in the case of intravenous administration of 8C7-toxin-expressing recombinant Bifidobacterium (2)>

Antitumor effect in the case of intravenous administration of 8C7-toxin-expressing recombinant *Bifidobacterium* to Xenograft model of a BxPC-3 cell was verified.

### (1) Measurement of the antitumor effects of recombinant Bifidobacterium in Xenograft model of BxPC-3 cell

2 × 10⁶ cells of BxPC-3 were subcutaneously transplanted into each 6-week-old female KSN/Slc nude mouse. After 12 days from transplantation, mice were grouped so that each group (Vehicle, 8C7 toxin, Toxin control; n = 5) had about an average tumor volume of 95 mm³. The 8C7-toxin-expressing recombinant *Bifidobacterium* prepared in Example 4 (1) and the Toxin-control-expressing recombinant *Bifidobacterium* prepared in Example 7 (1) were intravenously administered (1.5 × 10⁹ cells each). Furthermore, after 27 days from transplantation, 1.5×10⁹ cells of recombinant *Bifidobacterium* were intravenously administered again. As a negative control (Vehicle), a nude mouse that had intravenously received no recombinant *Bifidobacterium,* was used. As a nutritional supplement for *in vivo* recombinant *Bifidobacterium,* 1 mL of 20% lactulose (Wako Pure Chemical Industries, Ltd., Osaka, Japan) was intraperitoneally administered daily to all mice. Tumor size was measured using a vernier caliper on days 12, 15, 22, 27, 32, and 37 after subcutaneous transplantation of BxPC-3 cells. Tumor volume was calculated via the equation of "(shorter diameter)² × (longer diameter)/2".

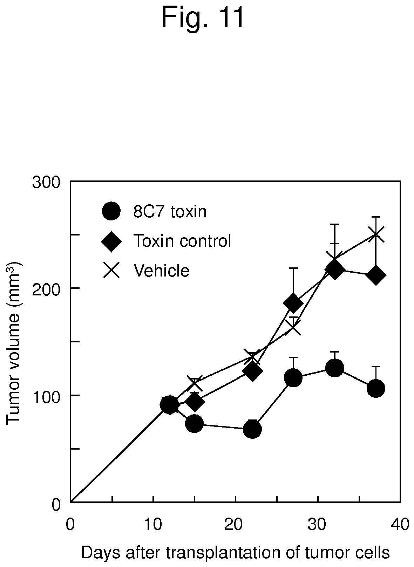

Results are shown in Fig. 11. 8C7-toxin-expressing recombinant *Bifidobacterium* was observed to exhibit the effect of suppressing tumor growth by 90%, compared with negative control (Vehicle). In contrast, almost no effect of suppressing tumor growth was observed for the recombinant *Bifidobacterium* expressing Toxin control as the negative control, containing no anti-EGFR single-chain antibody PMP8C7. These results suggest that, similar to Example 6, *in vivo,* the 8C7 toxin secreted from the recombinant *Bifidobacterium* was bound to the EGFR receptor on the surface of A431 cells via the anti-EGFR single-chain antibody moiety, and thus a cytotoxic effect was exhibited because of the *Pseudomonas aeruginosa* exotoxin A moiety. The antitumor effect obtained herein; that is, the effect of suppressing tumor growth was stronger than that of a combined use of an antimetabolite, gemcitabine that is used for treating pancreatic cancer and cetuximab that is a monoclonal antibody targeting EGFR (Clin Cancer Res. 2008; 14: 5142-5149) and thus is revealed to be sufficient as the effect of suppressing tumor growth.

### (2) Changes in body weight in Xenograft model of BxPC-3 cell upon administration of recombinant Bifidobacterium

Body weights of the nude mice to which the recombinant *Bifidobacterium* (1) had been administered were measured days 15 (Day 15), 22 (Day 22), 27 (Day 27), 32 (Day 32), and 37 (Day 37) after administration with the date on which the recombinant *Bifidobacterium* had been intravenously administered (Day 0) as the reference value. Measurement was performed for each group with n = 5.

The results are shown in Table 2.

**Table 2**

| Recombinant *Bifidobacterium* | Day 12 | Day 15 | Day 22 | Day 27 | Day 32 | Day 37 |
|---|---|---|---|---|---|---|
| Not administered | 1.00±0.04 | 1.09±0.02 | 1.12±0.02 | 1.17±0.02 | 1.14±0.02 | 1.17±0.02 |
| 8C7 Toxin | 1.00±0.03 | 1.08±0.02 | 1.08±0.02 | 1.10±0.03 | 1.12±0.03 | 1.13±0.03 |
| Toxin control | 1.00±0.02 | 1.10±0.03 | 1.12±0.02 | 1.15±0.02 | 1.18±0.01 | 1.19±0.02 |

No significant body weight loss due to intravenous administration of 8C7-Toxin-expressing recombinant *Bifidobacterium* was detected also for BxPC-3 cells, compared with Vehicle and Toxin control groups.

### <Example 8: Antitumor effects in the case of intravenous administration of 8C7-toxin-expressing recombinant Bifidobacterium (3)>

Antitumor effects when 8C7-toxin-expressing recombinant *Bifidobacterium* had been intravenously administered to Xenograft model of a PC-9 cell (human non-small cell lung cancer-derived cells obtained from Immuno-Biological Laboratories Co., Ltd., Fujioka-shi, Japan) were verified.

### (1) Measurement of the antitumor effects of recombinant Bifidobacterium in Xenograft model of PC-9 cell

1.7×10⁶ cells of PC-9 were subcutaneously transplanted into each 6-week-old female KSN/Slc nude mouse. After 8 days from transplantation, mice were grouped so that each group (Vehicle, 8C7 toxin, Toxin control; n = 5) had about an average tumor volume of 95 mm³. The 8C7-toxin-expressing recombinant *Bifidobacterium* prepared in Example 4 (1) and the Toxin-control-expressing recombinant *Bifidobacterium* prepared in Example 6 (1) were intravenously administered (1.5 × 10⁹ cells each). As a negative control (Vehicle), a nude mouse that had intravenously received no recombinant *Bifidobacterium,* was used. As a nutritional supplement for *in vivo* recombinant *Bifidobacterium,* 1 mL of 20% lactulose (Wako Pure Chemical Industries, Ltd., Osaka, Japan) was intraperitoneally administered daily to all mice. Tumor size was measured using a vernier caliper on days 9, 10, 11, 14, 17, 21, and 24 after subcutaneous transplantation of PC-9 cells. Tumor volume was calculated via the equation of "(shorter diameter)² × (longer diameter)/2".

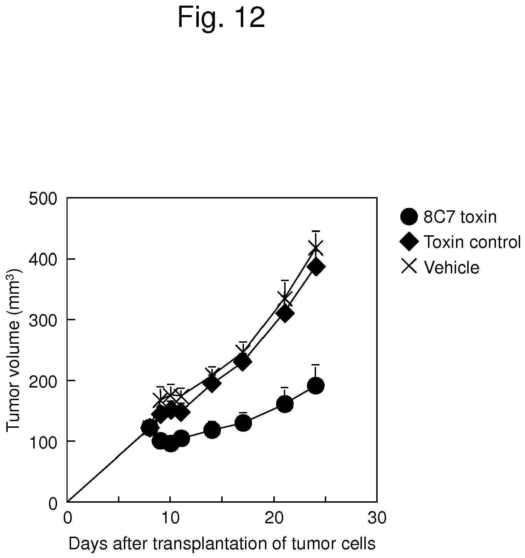

The results are shown in Fig. 12. 8C7-toxin-expressing recombinant *Bifidobacterium* was observed to exhibit the effect of suppressing tumor growth by 77%, compared with negative control (Vehicle). In contrast, almost no effect of suppressing tumor growth was observed for the recombinant *Bifidobacterium* expressing Toxin control as the negative control, containing no anti-EGFR single-chain antibody PMP8C7. These results suggest that, similar to Examples 6 and 7, *in vivo,* the 8C7 toxin secreted from the recombinant *Bifidobacterium* was bound to the EGFR receptor on the surface of A431 cells via the anti-EGFR single-chain antibody moiety, and thus a cytotoxic effect was exhibited because of the *Pseudomonas aeruginosa* exotoxin A moiety.

### <Example 9: Induction of intestinal immune response upon peroral administration of 8C7-EGFP-expressing recombinant Bifidobacterium>

Induction of intestinal immune response in the case of peroral administration of the 8C7-EGFP-expressing recombinant *Bifidobacterium* was verified.

### (1) Construction of pKKT427-containing recombinant Bifidobacterium as a negative control and peroral administration of 8C7-EGFP-expressing recombinant Bifidobacterium

pKKT427-containing recombinant *Bifidobacterium* as a negative control was prepared by introducing pKKT427 intact into *B. longum* 105-A using an electroporation method. Electroporation was performed under the conditions of 2.4 kV/cm, 25 µF, and 200Ω. The thus obtained recombinant *Bifidobacterium* and the 8C7-EGFP-expressing recombinant *Bifidobacterium* prepared in Example 5(1) were suspended in saline again, thereby preparing a solution to be administered to mice. 6.0 × 10⁹ cells of the recombinant *Bifidobacterium* were orally administered to each group (pKKT427, 8C7 toxin; n=4) of 6-week-old female BALB/cCrSlc mice (Japan SLC, Inc., Hamamatsu, Japan). As a nutritional supplement for intestinal recombinant *Bifidobacterium,* 0.2 mL of 20% lactulose was orally administered daily to all mice for 2 weeks. On day 31 after peroral administration of the recombinant *Bifidobacterium,* feces were collected, and then suspended in PBS containing ice-cooled complete mini (Roche Diagnostics, Basel, Swiss) to 20% (w/v). After centrifugation, the supernatant was filtered with a 0.22µm filter, to prepare a sample. To confirm the induction of intestinal immune response, antibody titer in feces against EGFP in each sample was measured by ELISA. ELISA was performed on a 96-well plate. Recombinant 8C7 EGFP (16 µg) prepared in Example 2 (2) was used as an antigen. After 1 or more hours of blocking at room temperature using Superblock blocking TBS (Thermo Fisher Scientific, Waltham, USA), the resultant was washed 3 times with TBS-T. 50 µL of the sample diluted 2-fold with Superblock blocking TBS was added. After 2 hours of incubation at room temperature, the resultant was washed 3 times with TBS-T. 50 µL of Goat polyclonal secondary antibody to mouse IgG+IgM+IgA-H&L HRP (abcam, Cambridge, UK) diluted 10000-fold was added. After 1 hour of incubation at 37°C, the resultant was washed 3 times with TBS-T. 50 µL of a TMB solution (Wako Pure Chemical Industries, Ltd., Osaka, Japan) was added. After 2 hours of incubation at room temperature, 50 µL of sulfuric acid, which was a coloring stop solution, was added, and then absorption at OD₄₅₀ was measured with an absorptiometer.

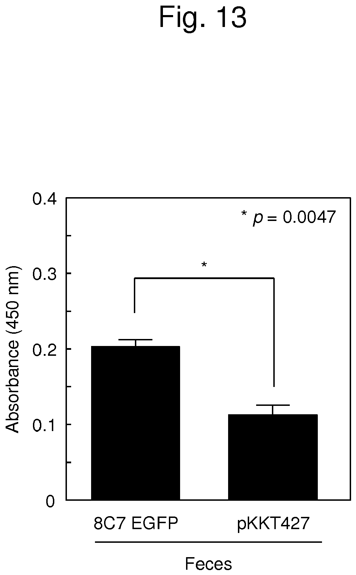

The results are shown in Fig 13. The 8C7-EGFP-expressing recombinant *Bifidobacterium* group exhibited significantly increased (t-test, p=0.0047) anti-EGFP antibody production in feces. This suggests that intestinal immune response was induced by the peroral administration of the 8C7-EGFP-expressing recombinant *Bifidobacterium.*

### <Example 10: Confirmation of no autoantibody production in blood in the case of intravenous administration of 8C7-EGFP-expressing recombinant Bifidobacterium>

It was confirmed that the intravenous administration of 8C7 EGFP-expressing recombinant *Bifidobacterium* did not induce any anti-8C7 EGFP antibody in the blood.

### (1) Construction of Xenograft model of colon26 cell and intravenous administration of recombinant Bifidobacterium

1.0 × 10⁶ cells of colon26 (mouse rectal cancer-derived cells, RIKEN BioResource Center, Tsukuba-shi, Japan) were subcutaneously transplanted into fifteen (15) 6-week-old female BALB/cCrSlc mice (Japan SLC, Inc., Hamamatsu, Japan). After 7 days from transplantation, mice were grouped into 3 groups (8C7 EGFP; n = 8, 8C7 EGFP (for collection of *Bifidobacterium*); n = 3, for untreated group; n = 4) so that an average tumor volume was 133 mm³. 1.0×10⁸ cells of the 8C7 EGFP-expressing recombinant *Bifidobacterium* were intravenously administered to each of the 8C7 EGFP group and the 8C7 EGFP (for collection of *Bifidobacterium)* group. The 8C7 EGFP (for collection of *Bifidobacterium*) group was a mouse group to be used in the next (2) for confirming the accumulation of 8C7-EGFP-expressing recombinant *Bifidobacterium* in tumors after administration thereof to mice to which colon26 cells had been subcutaneously transplanted. This 8C7 EGFP (for collection of *Bifidobacterium)* group undergone substantially the same treatment performed for the 8C7 EGFP group.

### (2) Accumulation of recombinant Bifidobacterium in tumors in the case of colon26 cells

Tumors were excised from the 8C7 EGFP (for collection of *Bifidobacterium*) group on day 18 after subcutaneous transplantation, homogenized in 1 mL of an MRS medium, and then applied to an MRS agar medium containing 100 µg/mL spectinomycin. Subsequently, a deoxidizer, Anaero Pack Kenki (Mitsubishi Gas Chemical Company, Inc., Tokyo, Japan) was added into a container or a bag to create a hypoxic condition allowing *Bifidobacterium* to grow, and then culture was performed under the thus created anaerobic environment at 37°C for 2 days. Colonies that had appeared on the MRS selective agar media were measured, and thus *Bifidobacterium* was obtained at an average of 4.3 × 10⁶ cells/mouse. It was confirmed by the results that the 8C7-EGFP-expressing recombinant *Bifidobacterium* intravenously administered to mice was accumulated in tumors in such a way that they could survive.

### (3) Confirmation of non-inductive nature of anti-8C7 EGFP antibody in serum

Serum was prepared from blood collected from each of 8C7-EGFP-expressing recombinant *Bifidobacterium* group and an untreated group on days 28 and 35 after subcutaneous transplantation. Antibody titer in serum against EGFP was measured by ELISA in order to examine the presence or the absence of the induction of immune response. ELISA was performed on a 96-well plate. The recombinant 8C7 EGFP (16 µg) prepared in Example 2 (2) was used as an antigen. After 1 or more hours of blocking at room temperature using Superblock blocking TBS, washing was performed 3 times with TBS-T. 50 µL of serum diluted 2000-fold with Superblock blocking TBS was added. As a negative control, 3 mg/mL Normal Mouse IgG (Invitrogen, Carlsbad, U.S.A.) was diluted 15000-fold with Superblock blocking TBS and then used. As a positive control, 1 mg/mL anti-EGFP antibody (Clontech, Mountain View, U.S.A.) was diluted 5000-fold with Superblock blocking TBS and then used. After 2 hours of incubation at room temperature, washing was performed 3 times with TBS-T. 50 µL of Goat polyclonal secondary antibody to mouse IgG+IgM+IgA-H&L HRP diluted 10000-fold was added, followed by 1 hour of incubation at 37°C. After washing was performed 3 times with TBS-T, 50 µL of a TMB solution was added. After 2 hours of incubation at room temperature, 50 µL of sulfuric acid, which was a coloring solution, was added, and then absorption at OD₄₅₀ was measured with an absorptiometer.

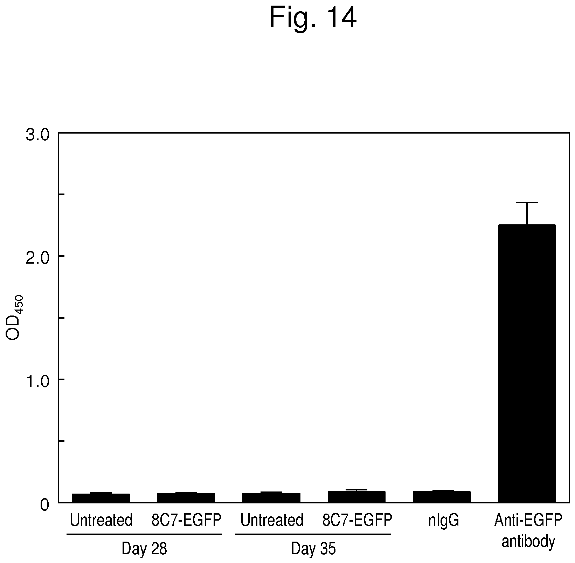

Results are shown in Fig. 14. The 8C7 EGFP-administered group (the group to which 8C7 EGFP had been administered) did not exhibit any significant increase in anti-EGFP antibody level in the serum, compared with those of the untreated group and the negative control nlgG. This suggests that even the intravenous administration of the 8C7-EGFP-expressing recombinant *Bifidobacterium* to Xenograft model of the colon26 cell induced no anti-8C7 EGFP antibody in the blood. When an immunotoxin is administered intravenously in clinical trials, autoantibody production will be a therapeutic problem (James A. Posey, Mohammad B. Khazaeli, Michael A. Bookman, et al, 2002, Clin Cancer Res, 8: 3092-3099). However, results demonstrate that autoantibody production will not be a problem in this therapeutic method.

### Industrial Applicability

According to the recombinant obligate anaerobic gram-positive *Bifidobacterium* of the present invention, a drug delivery carrier capable of delivering a nucleic acid encoding a functional peptide to regions around target cells, in an expressible and secretable state can be provided.

According to the antitumor agent and/or the marker for tumor detection of the present invention, an agent for suppressing solid cancer growth and/or a marker for tumor detection, which has low invasiveness, few side effects, and high target specificity, can be provided. The use of the agent for suppressing solid cancer growth and the marker for tumor detection makes it possible to monitor over time the therapeutic effects resulting from the suppression of solid cancer growth.

According to the oral vaccine agent described herein, a noninvasive oral vaccine agent having few side effects and high target specificity can be provided.

## Claims

1. A recombinant *Bifidobacterium,* comprising a nucleic acid encoding a fusion protein comprising a signal peptide, one or more V_{HH} antibodies of the family *Camelidae,* and one or more functional proteins selected from exotoxins, labeling proteins, enzymatic labels, or combinations thereof, in a secretable state,
wherein the V_{HH} antibodies of the family *Camelidae* recognize and bind to the surface antigen of a tumor cell.

2. The recombinant *Bifidobacterium* according to claim 1, wherein the nucleic acid encodes two or more V_{HH} antibodies of the family *Camelidae.*

3. The recombinant *Bifidobacterium* according to claim 1 or 2, wherein the labeling protein is a fluorescent protein or a luminescent protein.

4. The recombinant *Bifidobacterium* according to any one of claims 1 to 3, for use as an antitumor agent.

5. The recombinant *Bifidobacterium* for use according to claim 4, which is for use by systemic administration.

6. The recombinant *Bifidobacterium* for use according to claim 5, which is for use by intravascular injection.

7. The recombinant *Bifidobacterium* for use according to claim 6, which is for use by intravenous injection.

8. The recombinant *Bifidobacterium* for use according to any one of claims 5 to 7, which is for use by both systemic administration and direct administration to a tumor.

9. The recombinant *Bifidobacterium* according to any one of claims 1 to 3, for use *in vivo* for tumor detection.

## Patentansprüche

1. Rekombinantes Bifidobacterium, das eine Nucleinsäure umfasst, die für ein Fusionsprotein kodiert, das ein Signalpeptid, einen oder mehrere V_{HH}-Antikörper der Camelidae-Familie und ein oder mehrere funktionelle Proteine umfasst, die aus Exotoxinen, Markierungsproteinen, enzymatischen Markierungen oder Kombinationen davon ausgewählt sind, in einem sekretierbaren Zustand,
wobei die V_{HH}-Antikörper der Camelidae-Familie das Oberflächenantigen einer Tumorzelle erkennen und daran binden.

2. Rekombinantes Bifidobacterium nach Anspruch 1, wobei die Nucleinsäure für zwei oder mehr V_{HH}-Antikörper der Camelidae-Familie kodiert.

3. Rekombinantes Bifidobacterium nach Anspruch 1 oder 2, wobei das Markierungsprotein ein fluoreszierendes Protein oder lumineszierendes Protein ist.

4. Rekombinantes Bifidobacterium nach einem der Ansprüche 1 bis 3 zur Verwendung als Antitumormittel.

5. Rekombinantes Bifidobacterium zur Verwendung nach Anspruch 4, das zur systemischen Verabreichung dient.

6. Rekombinantes Bifidobacterium zur Verwendung nach Anspruch 5, das zur Verwendung mittels intravaskulärer Injektion dient.

7. Rekombinantes Bifidobacterium zur Verwendung nach Anspruch 6, das zur Verwendung mittels intravenöser Injektion dient.

8. Rekombinantes Bifidobacterium zur Verwendung nach einem der Ansprüche 5 bis 7, das zur Verwendung sowohl mittels systemischer Verabreichung als auch direkter Verabreichung an einen Tumor dient.

9. Rekombinantes Bifidobacterium nach einem der Ansprüche 1 bis 3 zur In-vivo-Verwendung bei der Tumordetektion.

## Revendications

1. *Bifidobacterium* recombinante, comprenant un acide nucléique codant pour une protéine de fusion comprenant un peptide signal, un ou plusieurs anticorps V_{HH} de la famille *Camelidae,* et une ou plusieurs protéines fonctionnelles sélectionnées parmi des exotoxines, des protéines de marquage, des marqueurs enzymatiques, ou des combinaisons de ceux-ci, dans un état sécrétable,
où les anticorps V_{HH} de la famille *Camelidae* reconnaissent et se lient à l'antigène de surface d'une cellule tumorale.

2. *Bifidobacterium* recombinante selon la revendication 1, dans laquelle l'acide nucléique code pour deux anticorps V_{HH} ou plus de la famille *Camelidae.*

3. *Bifidobacterium* recombinante selon la revendication 1 ou 2, dans laquelle la protéine de marquage est une protéine fluorescente ou une protéine luminescente.

4. *Bifidobacterium* recombinante selon l'une quelconque des revendications 1 à 3, destinée à être utilisée en tant qu'agent antitumoral.

5. *Bifidobacterium* recombinante destinée à être utilisée selon la revendication 4, qui est destinée à être utilisée par une administration systémique.

6. *Bifidobacterium* recombinante destinée à être utilisée selon la revendication 5, qui est destinée à être utilisée par une injection intravasculaire.

7. *Bifidobacterium* recombinante destinée à être utilisée selon la revendication 6, qui est destinée à être utilisée par une injection intraveineuse.

8. *Bifidobacterium* recombinante destinée à être utilisée selon l'une quelconque des revendications 5 à 7, qui est destinée à être utilisée à la fois par une administration systémique et une administration directe à une tumeur.

9. *Bifidobacterium* recombinante selon l'une quelconque des revendications 1 à 3, destinée à être utilisée *in vivo* pour la détection d'une tumeur.


### SEQUENCE LISTING

<110> Teikyo Heisei University
<120> Antitumor medicament, Marker for detecting tumor and Oral vaccine
<130> PH-5605-PCT
<150> JP 2012-158044
   <151> 2012-07-13
<160> 16
<170> PatentIn version 3.5
<210> 1
   <211> 2346
   <212> DNA
   <213> Artificial
<220>
   <223> synthetic DNA 8C7 toxin
<400> 1
   aagctttggg cgcggcggcc atgaagtggc ttgacaagca caatctcgtc tgatttttgc      60
   ccttgccctc ccctcgaaaa aaacacataa atcctatata aaatgcgggt tttcgcagtc     120
   acatgcgcta ttatcattga ttgaacgggc aaagcaacaa atgccgcccc ctgaccaaga     180
   aggatgcttt atgaccaacg ttcgcgtgat caagcccgct ctggcggccc tcgtggccgc     240
   cgccgcgtgc gtgggtgggc tggcctttag cagcgctcag cccgctcagg ccgacacgta     300
   ccaggtcaag ctggaggaaa gcggaggcgg gctggtccaa gctggcggtt ccctgcggct     360
   gtcctgcgcc gcctccggcc gtgcgttctc cagctacgtc atgggctggt ttcgccaggc     420
   gccgggcaag gagcgcgaga tcgtcggcgc catcagctgg cgcggcggga gcacctacta     480
   cgccgacagc gttaaggggc gcttcacgat cagccgcgac aacgcaaaga acactgtgta     540
   cctccagatg aattccctta agcccgagga caccgccgtc tactactgcg ccgcggtcta 600
   ccgcgtcggc gcgatttcgg agtatagcgg caccgactac tacaccgatg agtatgacta 660
   ttggggccag ggcacccagg tgaccgtttc gtccggtgga tccggcggtg gcggctcggg 720
   cgggcaagtg aaactggagg agtccggcgg aggtctggtg caagccggcg gctcgctccg 780
   gctcagctgt gccgcgtccg gccgcgcttt cagctcctat gtgatgggct ggttccgtca 840
   agccccgggc aaagagcggg agatcgtggg cgcgatctcc tggcgtggcg gttccaccta 900
   ttacgcagat tcggtcaagg gccgcttcac catctcgagg gacaacgcga agaacacggt 960
   ctatctccag atgaactccc tcaagccgga agataccgcc gtgtactact gcgcagccgt 1020
   gtaccgcgtg ggcgcaatct ccgagtacag cggaaccgat tactacaccg acgaatacga 1080
   ctactggggc caaggaaccc aggtgacggt ttccagcgag ggcggatccc tggccgccct 1140
   gacggcccac caggcctgcc accttcccct ggaaaccttc acgcgccacc gccaaccccg 1200
   tggctgggag cagctcgaac agtgcggcta tccggtgcag cggctggtcg ccctctacct 1260
   cgccgctcgc ctgtcctgga accaggtgga ccaggtgatc aggaacgctt tggctagccc 1320
   gggctccggc ggtgatctgg gtgaggccat ccgcgagcag cctgagcagg ctaggctggc 1380
   cttgaccttg gccgccgcag agtccgaacg cttcgtccgc cagggcaccg gaaacgatga 1440
   agccggggcc gcgaatgccg acgtggtgtc cctgacgtgt ccggtggccg caggcgagtg 1500
   cgccggacct gccgattccg gcgacgcact gctggagcgc aactacccga ccggcgcgga 1560
   attcctgggc gacggcggtg acgtgtcctt ctccacgagg ggaacgcaga actggactgt 1620
   cgagcggttg ctccaggccc atcgccagct ggaagagcgg ggttacgttt tcgtgggcta 1680
   ccacgggacc ttcctcgaag cggcgcagtc catcgtgttc ggcggggtgc gggcgaggtc 1740
   gcaggacctg gacgcgattt ggcgcggctt ctatatcgca ggcgaccccg ccctggcgta 1800
   cggttacgct caggaccagg agccggacgc gcgcgggagg atccgcaacg gtgccctcct 1860
   ccgcgtgtat gtcccgcgct cctcccttcc tggcttctac cgtacctccc tgacccttgc 1920
   cgcgccggaa gcggccggcg aagtggagcg tctgattggg catccgctgc cgttgcgcct 1980
   ggatgccatc actggcccgg aagaggaggg cggccggttg gaaacgatcc tcggctggcc 2040
   gctggcggaa cggacggtgg tcatcccgtc ggccatcccc accgacccgc gcaacgtcgg 2100
   cggcgacctg gatccgtcgt ccatcccgga taaggagcag gccatttcgg cgctgcccga 2160
   ctacgccagc cagccgggaa agccgccccg tgaggacctc aagtgaacta gtccttctgc 2220
   tcgtagcgat tacttcgagc attactgacg acaaagaccc cgaccgagat ggtcggggtc 2280
   tttttgttgt ggtgctgtga cgtgttgtcc aaccgtatta ttccggacta gatcagcggc 2340
   ggccgc 2346
<210> 2
   <211> 2025
   <212> DNA
   <213> Artificial
<220>
   <223> synthetic DNA 8C7EGFP
<400> 2
   aagctttggg cgcggcggcc atgaagtggc ttgacaagca caatctcgtc tgatttttgc 60
   ccttgccctc ccctcgaaaa aaacacataa atcctatata aaatgcgggt tttcgcagtc 120
   acatgcgcta ttatcattga ttgaacgggc aaagcaacaa atgccgcccc ctgaccaaga 180
   aggatgcttt atgaccaatg tgcgcgtcat caagcctgca ctggccgccc tggttgccgc 240
   cgccgcatgc gtgggcgggc tggcctttag cagcgcgcag ccggcccagg ctgacaccta 300
   tcaggtgaag ctggaagagt cgggtggcgg gttggtgcag gcgggcggct ccctccggtt     360
   gagctgcgct gcctcgggcc gcgccttcag ctcctacgtc atggggtggt tcaggcaggc     420
   gccgggcaag gaaagggaga tcgtcggggc catctcctgg aggggtggct ccacgtacta     480
   cgccgactcc gtgaaaggtc gcttcactat ctcgcgcgac aacgcgaaga acaccgtgta     540
   cctccagatg aactccctta agcccgaaga tacggcagtc tattactgcg cggcggtgta     600
   tcgcgtcggc gccatctccg agtactccgg caccgactac tacacggatg agtacgatta     660
   ctggggccag ggcacccagg tgacggtgag cagcggcggc agcggaggcg gcgggagcgg     720
   cggtcaagtg aagttggaag agtccggcgg cggcctggtc caagccggcg gcagcctccg     780
   tctgtcctgt gcggcctccg gacgtgcctt ctcgagctac gttatgggat ggtttcgcca     840
   ggctccgggg aaagaacgcg aaattgtggg cgccattagc tggcgcggcg gatccaccta     900
   ctacgccgat tcggttaagg gacgcttcac catctcccgt gacaacgcca agaacacggt     960
   gtatctgcag atgaacagcc tgaagccgga ggataccgcc gtctactact gcgctgcggt    1020
   ctatcgggtg ggcgcgatct ccgaatattc cggcaccgat tattacaccg acgagtatga    1080
   ctactggggc caaggtaccc aggtcacggt ttcctccggc ggttcgggcg gaggtggctc    1140
   cggcggtatg gtgtccaagg gcgaagagct gttcactggc gtggtgccga tcctggtgga    1200
   gctcgacggg gacgtgaatg gccacaaatt cagcgtgtcg ggcgaaggcg aaggcgacgc    1260
   cacctacggc aagctgacgc tgaaattcat ctgcaccacg ggcaaactcc ccgtcccgtg    1320
   gcccaccctg gtcacgactc tcacctacgg ggtccagtgc ttctcccgtt accctgacca    1380
   catgaagcaa cacgacttct tcaagtccgc gatgccggag ggctacgtgc aggaacgcac    1440
   gatcttcttc aaggacgacg gcaactacaa gacccgggcc gaggtcaagt tcgagggtga    1500
   cacccttgtg aatcgcatcg agctgaaggg aatcgacttc aaagaggacg gaaacattct 1560
   ggggcataag ctcgagtaca actacaactc gcacaacgtg tacatcatgg ctgacaagca 1620
   gaagaacggc atcaaggtga acttcaagat ccgccataac atcgaggatg gctccgtcca 1680
   gctcgcggac cattaccagc agaacacccc gattggcgat ggtcccgttc tcttgccgga 1740
   caaccactat ctgtcgacgc agtccgcgct gtccaaggat ccgaacgaga agcgggacca 1800
   catggtgctt ctcgagttcg tgaccgcagc cggcatcacc ctgggaatgg acgagctgta 1860
   caagcaccac catcaccacc attgaactag tccttctgct cgtagcgatt acttcgagca 1920
   ttactgacga caaagacccc gaccgagatg gtcggggtct ttttgttgtg gtgctgtgac 1980
   gtgttgtcca accgtattat tccggactag atcagcggcg gccgc 2025
<210> 3
   <211> 1539
   <212> DNA
   <213> Artificial
<220>
   <223> synthetic DNA Toxin control
<400> 3
   aagctttggg cgcggcggcc atgaagtggc ttgacaagca caatctcgtc tgatttttgc 60
   ccttgccctc ccctcgaaaa aaacacataa atcctatata aaatgcgggt tttcgcagtc 120
   acatgcgcta ttatcattga ttgaacgggc aaagcaacaa atgccgcccc ctgaccaaga 180
   aggatgcttt atgaccaacg tccgcgttat caagcctgcc ctcgcagcgt tggtggccgc 240
   agcggcgtgt gtgggcggac tggctttctc gtccgcacaa cccgcgcagg ctgagggcgg 300
   ctcgctggcg gcactcaccg cgcatcaggc gtgccatctg ccgctcgaaa ccttcacgcg 360
   tcacaggcaa ccgcgtgggt gggaacagct ggagcagtgc ggctatcccg tgcagaggct 420
   cgtcgctctg tacctggccg ccaggctgtc ctggaaccag gtggaccagg tcatccggaa 480
   cgcgcttgcc tccccgggct ccggcggcga tctgggcgag gccattcgcg aacagcccga 540
   gcaagcccgt ctggctctca cgctcgcagc cgcggagtcc gagcgcttcg tccgccaggg 600
   aaccggcaac gacgaggctg gtgcagccaa cgccgatgtc gtgtccctta cgtgcccggt 660
   tgccgccggc gaatgcgccg ggccggcgga cagcggcgac gcccttctgg aacgcaacta 720
   tccgaccggg gccgagtttc tcggcgatgg cggcgatgtg tccttctcca cccgcgggac 780
   ccagaactgg acggtcgagc gcctcctgca ggcccaccgc cagctcgagg agcgcggcta 840
   cgtgttcgtc gggtatcacg gcaccttcct ggaggccgcc cagagcatcg tgttcggtgg 900
   cgtgcgcgcc cgctcccagg acctggacgc gatctggcgt ggcttctaca tcgcgggtga 960
   tccggccctg gcctacggtt acgcccagga ccaggagcct gacgcccgtg gtcggatccg 1020
   caatggcgcg ctcctgaggg tctacgttcc tcgctcgtcc ctgccgggct tctaccgcac 1080
   ctccttgacc ctggccgccc cggaagccgc gggtgaagtg gagcggctga tcggacaccc 1140
   cttgccgctg cgcctggacg ccatcacggg cccggaagaa gagggcggac gcctcgagac 1200
   tatcctgggc tggccccttg ctgaacggac cgtggtgatt ccgagcgcca tcccgactga     1260
   cccgcggaac gtgggcggag atttggaccc gagcagcatt ccggacaagg agcaggcgat 1320
   cagcgctctg ccggactacg cctcgcaacc cggcaaaccg ccccgggagg acctgaagca 1380
   ccaccatcac catcactgaa ctagtccttc tgctcgtagc gattacttcg agcattactg 1440
   acgacaaaga ccccgaccga gatggtcggg gtctttttgt tgtggtgctg tgacgtgttg 1500
   tccaaccgta ttattccgga ctagatcagc ggcggccgc 1539
<210> 4
   <211> 184
   <212> DNA
   <213> Artificial
<220>
   <223> synthetic DNA derived from hup promoter of Bifidobacterium longum
<400> 4
   tgggcgcggc ggccatgaag tggcttgaca agcacaatct cgtctgattt ttgcccttgc 60
   cctcccctcg aaaaaaacac ataaatccta tataaaatgc gggttttcgc agtcacatgc 120
   gctattatca ttgattgaac gggcaaagca acaaatgccg ccccctgacc aagaaggatg 180
   cttt 184
<210> 5
   <211> 102
   <212> DNA
   <213> Artificial
<220>
   <223> synthetic DNA derived from usp secretory signal sequence of Bifidobacterium longum
<400> 5
   atgaccaacg ttcgcgtgat caagcccgct ctggcggccc tcgtggccgc cgccgcgtgc 60
   gtgggtgggc tggcctttag cagcgctcag cccgctcagg cc 102
<210> 6
   <211> 30
   <212> DNA
   <213> Artificial
<220>
   <223> synthetic DNA for linker
<400> 6
   ggtggatccg gcggtggcgg ctcgggcggg 30
<210> 7
   <211> 393
   <212> DNA
   <213> Artificial
<220>
   <223> synthetic DNA PMP8C7
<400> 7
   caggtcaagc tggaggaaag cggaggcggg ctggtccaag ctggcggttc cctgcggctg 60
   tcctgcgccg cctccggccg tgcgttctcc agctacgtca tgggctggtt tcgccaggcg 120
   ccgggcaagg agcgcgagat cgtcggcgcc atcagctggc gcggcgggag cacctactac 180
   gccgacagcg ttaaggggcg cttcacgatc agccgcgaca acgcaaagaa cactgtgtac 240
   ctccagatga attcccttaa gcccgaggac accgccgtct actactgcgc cgcggtctac 300
   cgcgtcggcg cgatttcgga gtatagcggc accgactact acaccgatga gtatgactat 360
   tggggccagg gcacccaggt gaccgtttcg tcc 393
<210> 8
   <211> 393
   <212> DNA
   <213> Artificial
<220>
   <223> synthetic DNA PMP8C7
<400> 8
   caagtgaaac tggaggagtc cggcggaggt ctggtgcaag ccggcggctc gctccggctc 60
   agctgtgccg cgtccggccg cgctttcagc tcctatgtga tgggctggtt ccgtcaagcc 120
   ccgggcaaag agcgggagat cgtgggcgcg atctcctggc gtggcggttc cacctattac 180
   gcagattcgg tcaagggccg cttcaccatc tcgagggaca acgcgaagaa cacggtctat 240
   ctccagatga actccctcaa gccggaagat accgccgtgt actactgcgc agccgtgtac 300
   cgcgtgggcg caatctccga gtacagcgga accgattact acaccgacga atacgactac 360
   tggggccaag gaacccaggt gacggtttcc agc 393
<210> 9
   <211> 1086
   <212> DNA
   <213> Artificial
<220>
   <223> synthetic DNA derived from exotoxin A
<400> 9
   gagggcggat ccctggccgc cctgacggcc caccaggcct gccaccttcc cctggaaacc 60
   ttcacgcgcc accgccaacc ccgtggctgg gagcagctcg aacagtgcgg ctatccggtg 120
   cagcggctgg tcgccctcta cctcgccgct cgcctgtcct ggaaccaggt ggaccaggtg 180
   atcaggaacg ctttggctag cccgggctcc ggcggtgatc tgggtgaggc catccgcgag 240
   cagcctgagc aggctaggct ggccttgacc ttggccgccg cagagtccga acgcttcgtc 300
   cgccagggca ccggaaacga tgaagccggg gccgcgaatg ccgacgtggt gtccctgacg 360
   tgtccggtgg ccgcaggcga gtgcgccgga cctgccgatt ccggcgacgc actgctggag 420
   cgcaactacc cgaccggcgc ggaattcctg ggcgacggcg gtgacgtgtc cttctccacg 480
   aggggaacgc agaactggac tgtcgagcgg ttgctccagg cccatcgcca gctggaagag 540
   cggggttacg ttttcgtggg ctaccacggg accttcctcg aagcggcgca gtccatcgtg 600
   ttcggcgggg tgcgggcgag gtcgcaggac ctggacgcga tttggcgcgg cttctatatc 660
   gcaggcgacc ccgccctggc gtacggttac gctcaggacc aggagccgga cgcgcgcggg 720
   aggatccgca acggtgccct cctccgcgtg tatgtcccgc gctcctccct tcctggcttc 780
   taccgtacct ccctgaccct tgccgcgccg gaagcggccg gcgaagtgga gcgtctgatt 840
   gggcatccgc tgccgttgcg cctggatgcc atcactggcc cggaagagga gggcggccgg 900
   ttggaaacga tcctcggctg gccgctggcg gaacggacgg tggtcatccc gtcggccatc 960
   cccaccgacc cgcgcaacgt cggcggcgac ctggatccgt cgtccatccc ggataaggag 1020
   caggccattt cggcgctgcc cgactacgcc agccagccgg gaaagccgcc ccgtgaggac 1080
   ctcaag 1086
<210> 10
   <211> 126
   <212> DNA
   <213> Artificial
<220>
   <223> synthetic DNA derived from HUT terminator
<400> 10
   ccttctgctc gtagcgatta cttcgagcat tactgacgac aaagaccccg accgagatgg 60
   tcggggtctt tttgttgtgg tgctgtgacg tgttgtccaa ccgtattatt ccggactaga 120
   tcagcg 126
<210> 11
   <211> 56
   <212> DNA
   <213> Artificial
<220>
   <223> primer
<400> 11
   cccatatgtg gtcccatccg cagtttgaaa aagacacgta ccaggtcaag ctggag 56
<210> 12
   <211> 44
   <212> DNA
   <213> Artificial
<220>
   <223> primer
<400> 12
   ttgcaagcaa ttgcggccgc cttcaggtcc tcccggggcg gttt 44
<210> 13
   <211> 56
   <212> DNA
   <213> Artificial
<220>
   <223> primer
<400> 13
   cccatatgtg gtcccatccg cagtttgaaa aagacaccta tcaggtgaag ctggaa 56
<210> 14
   <211> 44
   <212> DNA
   <213> Artificial
<220>
   <223> primer
<400> 14
   ttgcaagcaa ttgcggccgc cttgtacagc tcgtccattc ccag 44
<210> 15
   <211> 56
   <212> DNA
   <213> Artificial
<220>
   <223> primer
<400> 15
   cccatatgtg gtcccatccg cagtttgaaa aagagggcgg ctcgctggcg gcactc 56
<210> 16
   <211> 671
   <212> PRT
   <213> Artificial
<220>
   <223> synthetic amino acid
<400> 16
   Met Thr Asn Val Arg Val Ile Lys Pro Ala Leu Ala Ala Leu Val Ala
   1               5                   10                  15
   Ala Ala Ala Cys Val Gly Gly Leu Ala Phe Ser Ser Ala Gln Pro Ala
               20                  25                  30
   Gln Ala Asp Thr Tyr Gln Val Lys Leu Glu Glu Ser Gly Gly Gly Leu
           35                  40                  45
   Val Gln Ala Gly Gly Ser Leu Arg Leu Ser Cys Ala Ala Ser Gly Arg
       50                  55                  60
   Ala Phe Ser Ser Tyr Val Met Gly Trp Phe Arg Gln Ala Pro Gly Lys
   65                  70                  75                  80
   Glu Arg Glu Ile Val Gly Ala Ile Ser Trp Arg Gly Gly Ser Thr Tyr
   85 90 95
   Tyr Ala Asp Ser Val Lys Gly Arg Phe Thr Ile Ser Arg Asp Asn Ala
   100 105 110
   Lys Asn Thr Val Tyr Leu Gln Met Asn Ser Leu Lys Pro Glu Asp Thr
   115 120 125
   Ala Val Tyr Tyr Cys Ala Ala Val Tyr Arg Val Gly Ala Ile Ser Glu
   130 135 140
   Tyr Ser Gly Thr Asp Tyr Tyr Thr Asp Glu Tyr Asp Tyr Trp Gly Gln
   145 150 155 160
   Gly Thr Gln Val Thr Val Ser Ser Gly Gly Ser Gly Gly Gly Gly Ser
   165 170 175
   Gly Gly Gln Val Lys Leu Glu Glu Ser Gly Gly Gly Leu Val Gln Ala
   180 185 190
   Gly Gly Ser Leu Arg Leu Ser Cys Ala Ala Ser Gly Arg Ala Phe Ser
   195 200 205
   Ser Tyr Val Met Gly Trp Phe Arg Gln Ala Pro Gly Lys Glu Arg Glu
   210 215 220
   Ile Val Gly Ala Ile Ser Trp Arg Gly Gly Ser Thr Tyr Tyr Ala Asp
   225 230 235 240
   Ser Val Lys Gly Arg Phe Thr Ile Ser Arg Asp Asn Ala Lys Asn Thr
   245 250 255
   Val Tyr Leu Gln Met Asn Ser Leu Lys Pro Glu Asp Thr Ala Val Tyr
   260 265 270
   Tyr Cys Ala Ala Val Tyr Arg Val Gly Ala Ile Ser Glu Tyr Ser Gly
   275 280 285
   Thr Asp Tyr Tyr Thr Asp Glu Tyr Asp Tyr Trp Gly Gln Gly Thr Gln
   290 295 300
   Val Thr Val Ser Ser Glu Gly Gly Ser Leu Ala Ala Leu Thr Ala His
   305 310 315 320
   Gln Ala Cys His Leu Pro Leu Glu Thr Phe Thr Arg His Arg Gln Pro
   325 330 335
   Arg Gly Trp Glu Gln Leu Glu Gln Cys Gly Tyr Pro Val Gln Arg Leu
   340 345 350
   Val Ala Leu Tyr Leu Ala Ala Arg Leu Ser Trp Asn Gln Val Asp Gln
   355 360 365
   Val Ile Arg Asn Ala Leu Ala Ser Pro Gly Ser Gly Gly Asp Leu Gly
   370 375 380
   Glu Ala Ile Arg Glu Gln Pro Glu Gln Ala Arg Leu Ala Leu Thr Leu
   385 390 395 400
   Ala Ala Ala Glu Ser Glu Arg Phe Val Arg Gln Gly Thr Gly Asn Asp
   405 410 415
   Glu Ala Gly Ala Ala Asn Ala Asp Val Val Ser Leu Thr Cys Pro Val
   420 425 430
   Ala Ala Gly Glu Cys Ala Gly Pro Ala Asp Ser Gly Asp Ala Leu Leu
   435 440 445
   Glu Arg Asn Tyr Pro Thr Gly Ala Glu Phe Leu Gly Asp Gly Gly Asp
   450 455 460
   Val Ser Phe Ser Thr Arg Gly Thr Gln Asn Trp Thr Val Glu Arg Leu
   465 470 475 480
   Leu Gln Ala His Arg Gln Leu Glu Glu Arg Gly Tyr Val Phe Val Gly
   485 490 495
   Tyr His Gly Thr Phe Leu Glu Ala Ala Gln Ser Ile Val Phe Gly Gly
   500 505 510
   Val Arg Ala Arg Ser Gln Asp Leu Asp Ala Ile Trp Arg Gly Phe Tyr
   515 520 525
   Ile Ala Gly Asp Pro Ala Leu Ala Tyr Gly Tyr Ala Gln Asp Gln Glu
   530 535 540
   Pro Asp Ala Arg Gly Arg Ile Arg Asn Gly Ala Leu Leu Arg Val Tyr
   545 550 555 560
   Val Pro Arg Ser Ser Leu Pro Gly Phe Tyr Arg Thr Ser Leu Thr Leu
   565 570 575
   Ala Ala Pro Glu Ala Ala Gly Glu Val Glu Arg Leu Ile Gly His Pro
   580 585 590
   Leu Pro Leu Arg Leu Asp Ala Ile Thr Gly Pro Glu Glu Glu Gly Gly
   595 600 605
   Arg Leu Glu Thr Ile Leu Gly Trp Pro Leu Ala Glu Arg Thr Val Val
   610 615 620
   Ile Pro Ser Ala Ile Pro Thr Asp Pro Arg Asn Val Gly Gly Asp Leu
   625 630 635 640
   Asp Pro Ser Ser Ile Pro Asp Lys Glu Gln Ala Ile Ser Ala Leu Pro
   645 650 655
   Asp Tyr Ala Ser Gln Pro Gly Lys Pro Pro Arg Glu Asp Leu Lys
   660 665 670